# EUROPEAN PATENT APPLICATION

(11) **EP 4 744 675 A1**
(43) Date of publication of application: **20.05.2026**
(21) Application number: 24838877.9
(22) Date of filing: 11.07.2024
(51) Int. Cl.: A61K 38/17, C12N 15/85, C12N 15/62, A61K 45/06, A61P 25/00, A61P 9/10, A61P 9/14, A61P 19/00

(54) **COMPOSITION AND USE METHOD THEREFOR**

(30) Priority: 11.07.2023 CN 202310849960; 11.07.2023 CN 202310852222
(71) Applicant: Nanjing Reju Therapeutics Co., Ltd., Nanjing, Jiangsu 211199 (CN)
(72) Inventor: JI, Yin, Nanjing, Jiangsu 211199 (CN); WU, Yao, Nanjing, Jiangsu 211199 (CN); WANG, Xueping, Nanjing, Jiangsu 211199 (CN); ZHANG, Xiaodong, Nanjing, Jiangsu 211199 (CN)
(74) Representative: Rückerl, Florian
(86) International application number: PCT/CN2024/105026
(87) International publication number: WO 2025/011625

(57) **Abstract**

An aging reprogramming composition, a use method therefor, a kit containing the composition, and the use of the composition in tissue repair, tissue regeneration, organ regeneration, aging reversion, or any combination thereof. The present invention relates to SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1 and c-MYC, which are used as inducing factors to reprogram cells, tissues or organs to restore a youthful state. Provided is a composition of an activation inducing factor in the form of an engineered nucleic acid, an engineered cell, an engineered protein, a chemical agent, or any combination thereof, wherein the composition is used for treating diseases, preventing diseases, modulating tissue repair, modulating tissue regeneration, or any combination thereof.

## Description

### TECHNICAL FIELD

The present invention relates to an efficient method of inducing senescence reprogramming in somatic cells and simultaneously provides a reprogramming composition for rejuvenating a cell, tissue, or organ of a subject.

### BACKGROUND

All living things undergo aging accompanied by a decline in cell and tissue functions. Aging is an extremely complex process characterized by a time-dependent decline in function at the molecular, cellular, tissue, and organismal levels. Cellular senescence is not equivalent to cell death; senescent cells remain metabolically active over a period of time, but also shows some obvious changes: typical epigenetic alteration, genomic instability, telomere depletion, loss of protein homeostasis, mitochondrial dysfunction, stem cell depletion, altered intercellular communication, chronic inflammation, and a dozen other features.

Cell reprogramming technology refers to the regulation and alteration of the cell's fate through different ways, such as the introduction of exogenous gene, the treatment with small molecule compound, etc., without altering the genetic sequence of the cell. It also includes the improvement or even the reversal of the fate of cellular senescence. However, existing combinations of reprogramming factors that can achieve anti-aging or rejuvenate cells are more limited. Thus, there is an urgent need in the field for more combinations of reprogramming factors that are more effective in rejuvenating a cell, tissue, or organ of a subject or anti-aging.

### SUMMARY

The present invention relates to a composition for rejuvenating a cell, tissue, or organ of a subject, and a method for rejuvenating a cell, tissue, or organ of a subject using the composition, the composition and method being capable of restoring function of or rejuvenating a cell, tissue, or organ of a subject. The composition and method may be used in any disease or injury requiring repair or replacement of functionally failing tissue.

In a first aspect, the present invention provides a composition for rejuvenating a cell, tissue, or organ of a subject, the composition comprising: any one factor selected from the group consisting of SOX1, SOX2, SOX3, RCOR2, GMNN, and GLIS1; any one factor selected from the group consisting of GATA1, GATA2, GATA3, GATA4, GATA6, FOXA2, SOX7, SOX17, NKX3.1, NR5A2, C/EBPα, OCT4, and PAX1; factor Klf4 or Klf2; and/or, factor c-MYC.

In some embodiments, the composition comprises: any one factor selected from the group consisting of SOX1, SOX2, SOX3, RCOR2, and GMNN; any one factor selected from the group consisting of GATA1, GATA2, GATA3, GATA4, GATA6, FOXA2, SOX7, SOX17, NKX3.1, NR5A2, C/EBPα, OCT4, and PAX1; factor Klf4 or Klf2; and/or, factor c-MYC.

In some embodiments, the composition comprises: any one factor selected from the group consisting of GATA1, GATA2, GATA3, GATA4, GATA6, NKX3.1, FOXA2, NR5A2, OCT4, and SOX7; factor KLF2 or KLF4; any one factor selected from the group consisting of SOX1, SOX2, GLIS1, and SOX3; and/or, factor c-MYC.

In some embodiments, the composition comprises GATA6, SOX3, and Klf4.

In some embodiments, the composition comprises OCT4, SOX3, and Klf4.

In some embodiments, the composition comprises PAX1, SOX3, and Klf4.

In some embodiments, the composition comprises GATA3, SOX3, and Klf4.

In some embodiments, the composition comprises C/EBPα, SOX3, and Klf4.

In some embodiments, the composition comprises OCT4, SOX1, and Klf4.

In some embodiments, the composition comprises GATA3, SOX1, and Klf4.

In some embodiments, the composition comprises GATA6, SOX1, and Klf4.

In some embodiments, the composition comprises SOX17, SOX1, and Klf4.

In some embodiments, the composition comprises NR5A2, SOX2, and Klf4.

In some embodiments, the composition comprises GATA1, SOX2, and Klf4.

In some embodiments, the composition comprises GATA2, SOX2, and Klf4.

In some embodiments, the composition comprises GATA3, SOX2, and Klf4.

In some embodiments, the composition comprises GATA4, SOX2, and Klf4.

In some embodiments, the composition comprises GATA6, SOX2, and Klf4.

In some embodiments, the composition comprises FOXA2, SOX2, and Klf4.

In some embodiments, the composition comprises SOX7, SOX2, and Klf4.

In some embodiments, the composition comprises PAX1, SOX2, and Klf4.

In some embodiments, the composition comprises SOX17, SOX2, and Klf4.

In some embodiments, the composition comprises C/EBPα, SOX2, and Klf4.

In some embodiments, the composition comprises NKX3.1, SOX2, and Klf4.

In some embodiments, the composition comprises GATA6, GMNN, and Klf4.

In some embodiments, the composition comprises OCT4, RCOR2, and Klf4.

In some embodiments, the composition comprises OCT4, SOX2, and Klf2.

In some embodiments, the composition comprises OCT4, GMNN, and Klf2.

In some embodiments, the composition comprises OCT4, RCOR2, and Klf2.

In some embodiments, the composition comprises FOXA2, SOX2, and Klf2.

In some embodiments, the composition comprises GATA1, SOX1, and KLF4.

In some embodiments, the composition comprises GATA2, SOX1, and KLF4.

In some embodiments, the composition comprises GATA4, SOX1, and KLF4.

In some embodiments, the composition comprises FOXA2, SOX1, and KLF4.

In some embodiments, the composition comprises NKX3.1, SOX1, and KLF4.

In some embodiments, the composition comprises GATA1, SOX3, and KLF4.

In some embodiments, the composition comprises GATA2, SOX3, and KLF4.

In some embodiments, the composition comprises GATA4, SOX3, and KLF4.

In some embodiments, the composition comprises FOXA2, SOX3, and KLF4.

In some embodiments, the composition comprises NKX3.1, SOX3, and KLF4.

In some embodiments, the composition comprises SOX7, SOX3, and KLF4.

In some embodiments, the composition comprises NR5A2, SOX3, and KLF4.

In some embodiments, the composition comprises GATA1, SOX1, and KLF2.

In some embodiments, the composition comprises GATA2, SOX1, and KLF2.

In some embodiments, the composition comprises GATA3, SOX1, and KLF2.

In some embodiments, the composition comprises GATA4, SOX1, and KLF2.

In some embodiments, the composition comprises GATA6, SOX1, and KLF2.

In some embodiments, the composition comprises NKX3.1, SOX1, and KLF2.

In some embodiments, the composition comprises OCT4, SOX1, and KLF2.

In some embodiments, the composition comprises GATA3, SOX2, and KLF2.

In some embodiments, the composition comprises GATA4, SOX2, and KLF2.

In some embodiments, the composition comprises GATA6, SOX2, and KLF2.

In some embodiments, the composition comprises GATA1, SOX3, and KLF2.

In some embodiments, the composition comprises GATA2, SOX3, and KLF2.

In some embodiments, the composition comprises GATA6, SOX3, and KLF2.

In some embodiments, the composition comprises OCT4, SOX3, and KLF2.

In some embodiments, the composition comprises KLF4, OCT4, and GLIS1.

In some embodiments, the composition comprises KLF2, OCT4, and GLIS1.

In some embodiments, the composition comprises GATA6, SOX3, Klf4, and c-MYC.

In some embodiments, the composition comprises GATA3, SOX3, Klf4, and c-MYC.

In some embodiments, the composition comprises OCT4, SOX3, Klf4, and c-MYC.

In some embodiments, the composition comprises PAX1, SOX3, Klf4, and c-MYC.

In some embodiments, the composition comprises C/EBPα, SOX3, Klf4, and c-MYC.

In some embodiments, the composition comprises OCT4, SOX1, Klf4, and c-MYC.

In some embodiments, the composition comprises GATA3, SOX1, Klf4, and c-MYC.

In some embodiments, the composition comprises GATA6, SOX1, Klf4, and c-MYC.

In some embodiments, the composition comprises SOX17, SOX1, Klf4, and c-MYC.

In some embodiments, the composition comprises NR5A2, SOX2, Klf4, and c-MYC.

In some embodiments, the composition comprises GATA1, SOX2, Klf4, and c-MYC.

In some embodiments, the composition comprises GATA2, SOX2, Klf4, and c-MYC.

In some embodiments, the composition comprises GATA3, SOX2, Klf4, and c-MYC.

In some embodiments, the composition comprises GATA4, SOX2, Klf4, and c-MYC.

In some embodiments, the composition comprises GATA6, SOX2, Klf4, and c-MYC.

In some embodiments, the composition comprises FOXA2, SOX2, Klf4, and c-MYC.

In some embodiments, the composition comprises SOX7, SOX2, Klf4, and c-MYC.

In some embodiments, the composition comprises PAX1, SOX2, Klf4, and c-MYC.

In some embodiments, the composition comprises SOX17, SOX2, Klf4, and c-MYC.

In some embodiments, the composition comprises C/EBPα, SOX2, Klf4, and c-MYC.

In some embodiments, the composition comprises NKX3.1, SOX2, Klf4, and c-MYC.

In some embodiments, the composition comprises GATA6, GMNN, Klf4, and c-MYC.

In some embodiments, the composition comprises OCT4, RCOR2, Klf4, and c-MYC.

In some embodiments, the composition comprises OCT4, SOX2, Klf2, and c-MYC.

In some embodiments, the composition comprises OCT4, GMNN, Klf2, and c-MYC.

In some embodiments, the composition comprises OCT4, RCOR2, Klf2, and c-MYC.

In some embodiments, the composition comprises FOXA2, SOX2, Klf2, and c-MYC.

In some embodiments, the composition comprises GATA1, SOX1, KLF4, and c-MYC.

In some embodiments, the composition comprises GATA2, SOX1, KLF4, and c-MYC.

In some embodiments, the composition comprises GATA4, SOX1, KLF4, and c-MYC.

In some embodiments, the composition comprises FOXA2, SOX1, KLF4, and c-MYC.

In some embodiments, the composition comprises NKX3.1, SOX1, KLF4, and c-MYC.

In some embodiments, the composition comprises GATA1, SOX3, KLF4, and c-MYC.

In some embodiments, the composition comprises GATA2, SOX3, KLF4, and c-MYC.

In some embodiments, the composition comprises GATA4, SOX3, KLF4, and c-MYC.

In some embodiments, the composition comprises FOXA2, SOX3, KLF4, and c-MYC.

In some embodiments, the composition comprises NKX3.1, SOX3, KLF4, and c-MYC.

In some embodiments, the composition comprises SOX7, SOX3, KLF4, and c-MYC.

In some embodiments, the composition comprises NR5A2, SOX3, KLF4, and c-MYC.

In some embodiments, the composition comprises GATA1, SOX1, KLF2, and c-MYC.

In some embodiments, the composition comprises GATA2, SOX1, KLF2, and c-MYC.

In some embodiments, the composition comprises GATA3, SOX1, KLF2, and c-MYC.

In some embodiments, the composition comprises GATA4, SOX1, KLF2, and c-MYC.

In some embodiments, the composition comprises GATA6, SOX1, KLF2, and c-MYC.

In some embodiments, the composition comprises NKX3.1, SOX1, KLF2, and c-MYC.

In some embodiments, the composition comprises OCT4, SOX1, KLF2, and c-MYC.

In some embodiments, the composition comprises GATA3, SOX2, KLF2, and c-MYC.

In some embodiments, the composition comprises GATA4, SOX2, KLF2, and c-MYC.

In some embodiments, the composition comprises GATA6, SOX2, KLF2, and c-MYC.

In some embodiments, the composition comprises GATA1, SOX3, KLF2, and c-MYC.

In some embodiments, the composition comprises GATA2, SOX3, KLF2, and c-MYC.

In some embodiments, the composition comprises GATA6, SOX3, KLF2, and c-MYC.

In some embodiments, the composition comprises OCT4, SOX3, KLF2, and c-MYC.

In some embodiments, the composition comprises KLF4, OCT4, GLIS1, and c-MYC.

In some embodiments, the composition comprises KLF2, OCT4, GLIS1, and c-MYC.

In some embodiments, the factor is a protein, a nucleic acid encoding the same, or a mixture thereof.

For example, the SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC is a protein, a fusion protein bound to a transmembrane domain, or a nucleic acid encoding the same, e.g., in the form of DNA or RNA.

In some embodiments, the factor is provided in the form of an expression vector, and the expression vector may be a plasmid vector, an add-on vector, a transposon, or a virus-derived vector such as an adenovirus vector, an adeno-associated virus vector (AAV), a lentivirus vector, a Sendai virus vector, a cytomegalovirus vector, or a cowpox virus vector.

In some embodiments, the factors are present on one or more expression vectors.

In some embodiments, the factors are present on a same expression vector.

In some embodiments, the factor is in the form of mRNA.

In some embodiments, the factors are linked in tandem on the same mRNA molecule, or each of the factors is on a separate mRNA molecule, wherein the factors are in the form of a complex of multiple mRNA molecules.

In some embodiments, the factor is a synthetic mRNA, and the synthetic mRNA encodes a wild-type form, a mutant that retains activity thereof, or a genetically engineered form of any one selected from the group consisting of SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, and c-MYC.

In some embodiments, the composition further comprises a pharmaceutically acceptable carrier or adjuvant.

In some embodiments, the subject is a mammal including, but not limited to, human, mouse, rat, bovine, ovine, equine, canine, feline, porcine, or monkey.

In some embodiments, the subject is chicken, duck, goose, or bird.

In some embodiments, the cell, tissue, or organ of a subject is derived from, but not limited to, eye, ear, nose, or mouth including gum and root; bone, lung, breast, pancreas, stomach, or esophagus; muscle, including cardiac muscle; liver, or blood vessel; skin, including hair; heart, brain, neural tissue, kidney, testis, prostate, penis, cloaca, fin, ovary, or intestine.

In some embodiments, the composition for rejuvenating a cell, tissue, or organ of a subject can restore epigenetic information in the cell, tissue, or organ of the subject.

In some embodiments, the composition for rejuvenating a cell, tissue, or organ of a subject can restore loss of epigenetic information due to aging, injury, or disease in the cell, tissue, or organ of the subject.

In some embodiments, a promoter may be used to regulate the expression of a factor to reduce and reverse epigenetic marks associated with aging, increase epigenetic marks associated with cellular rejuvenation, reduce the expression of aging-associated protein, increase the expression of protein associated with cellular rejuvenation, restore the balance between euchromatin and heterochromatin, prevent the loss of cellular identity, restore cellular identity, and reverse aging-associated DNA methylation change, thereby rejuvenating the cell.

In some embodiments, the SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC is a human protein, or a non-human protein, e.g., a protein from a mammal (e.g., mouse, rat, bovine, ovine, equine, canine, feline, porcine, monkey, chicken, duck, goose, and bird), or a protein having at least 80%, at least 85%, at least 90%, at least 95%, at least 96%, at least 97%, at least 98%, at least 99%, or 100% identity to the amino acid sequence of the corresponding human or non-human protein and maintaining activity.

In some embodiments, the SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, c-MYC, or any combination thereof is a nucleic acid encoding a human protein or a non-human protein, e.g., a nucleic acid encoding a protein from a mammal (e.g., mouse, rat, bovine, ovine, equine, canine, feline, porcine, monkey, chicken, duck, goose, and bird) or a nucleic acid having at least 80%, at least 85%, at least 90%, at least 95%, at least 96%, at least 97%, at least 98%, at least 99%, or 100% identity to the nucleic acid sequence of the corresponding nucleic acid encoding a human protein or a non-human protein, and maintaining activity.

In some embodiments, the rejuvenating a cell, tissue, or organ of a subject comprises restoring lost transcriptional profile or epigenetic information in the at least one cell, tissue, or organ due to aging, injury, disease, or any combination thereof.

In some embodiments, the rejuvenating a cell, tissue, or organ of a subject comprises restoring the function of the cell, increasing the potential of the cell, enhancing the viability of the cell, or increasing the replicative capacity or lifespan of the cell, or a combination thereof.

In some embodiments, the rejuvenating a cell, tissue, or organ of a subject comprises promoting axonal regeneration of a neuronal cell in a subject; reversing the epigenetic age of a skin fibroblast in a subject; reversing senescence of a skin fibroblast in a subject; reversing senescence of a chondrocyte in a subject; reversing senescence of a muscle stem cell in a subject; promoting improvement of osteoarthritis symptoms in a subject; or promoting improvement of ocular disease symptoms in a subject.

In some embodiments, the subject suffers from, is suspected of suffering from, or is at risk of suffering from:
a disease, condition, or symptom associated with impaired cell, tissue, or organ viability, or with aging;
the disease, condition, or symptom associated with impaired cell, tissue, or organ viability, or with aging is a neurodegenerative disease, cardiovascular disease, metabolic disease, musculoskeletal disease, inflammatory disease, symptom or condition associated with the skin, ocular disease, fibrotic disease, or disease associated with the reproductive system;
the neurodegenerative disease is stroke, Huntington's chorea, dementia, Alzheimer's disease, or Parkinson's disease;
the cardiovascular disease is hypertension, coronary arrhythmia, heart disease, hypotension, heart failure, angina pectoris, arteriosclerosis, myocardial necrosis, myocarditis, congestive heart failure, atrioventricular block, atherosclerosis, or myocardial infarction;
the metabolic disease is type I diabetes, type II diabetes, hyperglycemia, hyperinsulinemia, insulin resistance, obesity, or gout;
the musculoskeletal disease is muscular dystrophy, myasthenia, myasthenia gravis, osteoporosis, osteoarthritis, osteochondrosis, osteochondritis, osteonecrosis, or osteopenia;
the inflammatory disease is systemic lupus erythematosus, rheumatic polymyalgia, gouty arthritis, degenerative arthritis, rheumatoid arthritis, inflammatory arthritis, Hashimoto's thyroiditis, inflammatory bowel disease, hepatitis, pneumonia, respiratory inflammation, or encephalitis;
the symptom or condition associated with the skin is wrinkles, age spots, seborrheic keratosis, tinea manuum and pedis, urticaria, neurodermatitis, or pigmented nevi;
the ocular disease is macular degeneration, retinal degeneration, retinal detachment, diabetic retinopathy, glaucoma, optic atrophy, floaters, cataract, non-arteritic anterior ischemic optic neuropathy, chorioretinitis, retinitis pigmentosa, or optic nerve injury;
the fibrotic disease is pulmonary fibrosis, hepatic fibrosis, myocardial fibrosis, renal interstitial fibrosis, myelofibrosis, pleural fibrosis, or intestinal wall fibrosis; and
the disease associated with the reproductive system is erectile dysfunction or premature ovarian failure.

In some embodiments, the composition induces cell reprogramming, reverses aging, improves tissue function, improves organ function, promotes tissue repair, promotes tissue survival, promotes tissue regeneration, promotes tissue growth, promotes angiogenesis, reduces scar formation, attenuates the external manifestations of aging including alopecia areata, thinning of hair, graying of hair, sagging of the skin, and wrinkling of the skin, promotes organ regeneration, promotes organ survival, treatment of diseases or any combination thereof.

In some embodiments, the composition reverses the aging of a cell, tissue, organ, or a subject, thereby rejuvenating the cell, tissue, or organ.

In some embodiments, the composition may promote neuronal regeneration in a subject.

In some embodiments, the composition may protect neurons in a subject from damage or degeneration.

In some embodiments, the composition may reverse the epigenetic age of a skin fibroblast in a subject.

In some embodiments, the composition may reverse senescence of a skin fibroblast in a subject.

In some embodiments, the composition may reverse senescence of a chondrocyte in a subject.

In some embodiments, the composition may reverse senescence of a muscle stem cell in a subject.

In some embodiments, the composition may promote improvement of osteoarthritis symptom in a subject.

In some embodiments, the composition may promote improvement of glaucoma symptom in a subject.

In some embodiments, the composition may promote regeneration of optic nerve.

In some embodiments, the composition may promote axonal regeneration of optic nerve.

In some embodiments, the composition may inhibit apoptosis of optic nerve cells (e.g., retinal ganglion cells) in the optic nerve injury.

In some embodiments, the composition does not induce teratoma formation or does not induce tumor growth or tumor formation.

In some embodiments, the composition does not induce complete reprogramming.

In some embodiments, the composition does not reprogram a cell into an induced pluripotent stem cell (iPSC).

In a second aspect, the present invention provides a method for rejuvenating a cell, tissue, or organ of a subject, the method comprising administering the composition of the first aspect into the subject.

In some embodiments, the composition comprises: any one factor selected from the group consisting of SOX1, SOX2, SOX3, RCOR2, GMNN, and GLIS1; any one factor selected from the group consisting of GATA1, GATA2, GATA3, GATA4, GATA6, FOXA2, SOX7, SOX17, NKX3.1, NR5A2, C/EBPα, OCT4, and PAX1; factor Klf4 or Klf2; and/or, factor c-MYC.

In some embodiments, the composition comprises: any one factor selected from the group consisting of SOX1, SOX2, SOX3, RCOR2, and GMNN; any one factor selected from the group consisting of GATA1, GATA2, GATA3, GATA4, GATA6, FOXA2, SOX7, SOX17, NKX3.1, NR5A2, C/EBPα, OCT4, and PAX1; factor Klf4 or Klf2; and/or, factor c-MYC.

In some embodiments, the composition comprises: any one factor selected from the group consisting of GATA1, GATA2, GATA3, GATA4, GATA6, NKX3.1, FOXA2, NR5A2, OCT4, and SOX7; factor KLF2 or KLF4; any one factor selected from the group consisting of SOX1, SOX2, GLIS1, and SOX3; and/or, factor c-MYC.

In some embodiments, the composition comprises GATA6, SOX3, and Klf4.

In some embodiments, the composition comprises OCT4, SOX3, and Klf4.

In some embodiments, the composition comprises PAX1, SOX3, and Klf4.

In some embodiments, the composition comprises GATA3, SOX3, and Klf4.

In some embodiments, the composition comprises C/EBPα, SOX3, and Klf4.

In some embodiments, the composition comprises OCT4, SOX1, and Klf4.

In some embodiments, the composition comprises GATA3, SOX1, and Klf4.

In some embodiments, the composition comprises GATA6, SOX1, and Klf4.

In some embodiments, the composition comprises SOX17, SOX1, and Klf4.

In some embodiments, the composition comprises NR5A2, SOX2, and Klf4.

In some embodiments, the composition comprises GATA1, SOX2, and Klf4.

In some embodiments, the composition comprises GATA2, SOX2, and Klf4.

In some embodiments, the composition comprises GATA3, SOX2, and Klf4.

In some embodiments, the composition comprises GATA4, SOX2, and Klf4.

In some embodiments, the composition comprises GATA6, SOX2, and Klf4.

In some embodiments, the composition comprises FOXA2, SOX2, and Klf4.

In some embodiments, the composition comprises SOX7, SOX2, and Klf4.

In some embodiments, the composition comprises PAX1, SOX2, and Klf4.

In some embodiments, the composition comprises SOX17, SOX2, and Klf4.

In some embodiments, the composition comprises C/EBPα, SOX2, and Klf4.

In some embodiments, the composition comprises NKX3.1, SOX2, and Klf4.

In some embodiments, the composition comprises GATA6, GMNN, and Klf4.

In some embodiments, the composition comprises OCT4, RCOR2, and Klf4.

In some embodiments, the composition comprises OCT4, SOX2, and Klf2.

In some embodiments, the composition comprises OCT4, GMNN, and Klf2.

In some embodiments, the composition comprises OCT4, RCOR2, and Klf2.

In some embodiments, the composition comprises FOXA2, SOX2, and Klf2.

In some embodiments, the composition comprises GATA1, SOX1, and KLF4.

In some embodiments, the composition comprises GATA2, SOX1, and KLF4.

In some embodiments, the composition comprises GATA4, SOX1, and KLF4.

In some embodiments, the composition comprises FOXA2, SOX1, and KLF4.

In some embodiments, the composition comprises NKX3.1, SOX1, and KLF4.

In some embodiments, the composition comprises GATA1, SOX3, and KLF4.

In some embodiments, the composition comprises GATA2, SOX3, and KLF4.

In some embodiments, the composition comprises GATA4, SOX3, and KLF4.

In some embodiments, the composition comprises FOXA2, SOX3, and KLF4.

In some embodiments, the composition comprises NKX3.1, SOX3, and KLF4.

In some embodiments, the composition comprises SOX7, SOX3, and KLF4.

In some embodiments, the composition comprises NR5A2, SOX3, and KLF4.

In some embodiments, the composition comprises GATA1, SOX1, and KLF2.

In some embodiments, the composition comprises GATA2, SOX1, and KLF2.

In some embodiments, the composition comprises GATA3, SOX1, and KLF2.

In some embodiments, the composition comprises GATA4, SOX1, and KLF2.

In some embodiments, the composition comprises GATA6, SOX1, and KLF2.

In some embodiments, the composition comprises NKX3.1, SOX1, and KLF2.

In some embodiments, the composition comprises OCT4, SOX1, and KLF2.

In some embodiments, the composition comprises GATA3, SOX2, and KLF2.

In some embodiments, the composition comprises GATA4, SOX2, and KLF2.

In some embodiments, the composition comprises GATA6, SOX2, and KLF2.

In some embodiments, the composition comprises GATA1, SOX3, and KLF2.

In some embodiments, the composition comprises GATA2, SOX3, and KLF2.

In some embodiments, the composition comprises GATA6, SOX3, and KLF2.

In some embodiments, the composition comprises OCT4, SOX3, and KLF2.

In some embodiments, the composition comprises KLF4, OCT4, and GLIS1.

In some embodiments, the composition comprises KLF2, OCT4, and GLIS1.

In some embodiments, the composition comprises GATA6, SOX3, Klf4, and c-MYC.

In some embodiments, the composition comprises GATA3, SOX3, Klf4, and c-MYC.

In some embodiments, the composition comprises OCT4, SOX3, Klf4, and c-MYC.

In some embodiments, the composition comprises PAX1, SOX3, Klf4, and c-MYC.

In some embodiments, the composition comprises C/EBPα, SOX3, Klf4, and c-MYC.

In some embodiments, the composition comprises OCT4, SOX1, Klf4, and c-MYC.

In some embodiments, the composition comprises GATA3, SOX1, Klf4, and c-MYC.

In some embodiments, the composition comprises GATA6, SOX1, Klf4, and c-MYC.

In some embodiments, the composition comprises SOX17, SOX1, Klf4, and c-MYC.

In some embodiments, the composition comprises NR5A2, SOX2, Klf4, and c-MYC.

In some embodiments, the composition comprises GATA1, SOX2, Klf4, and c-MYC.

In some embodiments, the composition comprises GATA2, SOX2, Klf4, and c-MYC.

In some embodiments, the composition comprises GATA3, SOX2, Klf4, and c-MYC.

In some embodiments, the composition comprises GATA4, SOX2, Klf4, and c-MYC.

In some embodiments, the composition comprises GATA6, SOX2, Klf4, and c-MYC.

In some embodiments, the composition comprises FOXA2, SOX2, Klf4, and c-MYC.

In some embodiments, the composition comprises SOX7, SOX2, Klf4, and c-MYC.

In some embodiments, the composition comprises PAX1, SOX2, Klf4, and c-MYC.

In some embodiments, the composition comprises SOX17, SOX2, Klf4, and c-MYC.

In some embodiments, the composition comprises C/EBPα, SOX2, Klf4, and c-MYC.

In some embodiments, the composition comprises NKX3.1, SOX2, Klf4, and c-MYC.

In some embodiments, the composition comprises GATA6, GMNN, Klf4, and c-MYC.

In some embodiments, the composition comprises OCT4, RCOR2, Klf4, and c-MYC.

In some embodiments, the composition comprises OCT4, SOX2, Klf2, and c-MYC.

In some embodiments, the composition comprises OCT4, GMNN, Klf2, and c-MYC.

In some embodiments, the composition comprises OCT4, RCOR2, Klf2, and c-MYC.

In some embodiments, the composition comprises FOXA2, SOX2, Klf2, and c-MYC.

In some embodiments, the composition comprises GATA1, SOX1, KLF4, and c-MYC.

In some embodiments, the composition comprises GATA2, SOX1, KLF4, and c-MYC.

In some embodiments, the composition comprises GATA4, SOX1, KLF4, and c-MYC.

In some embodiments, the composition comprises FOXA2, SOX1, KLF4, and c-MYC.

In some embodiments, the composition comprises NKX3.1, SOX1, KLF4, and c-MYC.

In some embodiments, the composition comprises GATA1, SOX3, KLF4, and c-MYC.

In some embodiments, the composition comprises GATA2, SOX3, KLF4, and c-MYC.

In some embodiments, the composition comprises GATA4, SOX3, KLF4, and c-MYC.

In some embodiments, the composition comprises FOXA2, SOX3, KLF4, and c-MYC.

In some embodiments, the composition comprises NKX3.1, SOX3, KLF4, and c-MYC.

In some embodiments, the composition comprises SOX7, SOX3, KLF4, and c-MYC.

In some embodiments, the composition comprises NR5A2, SOX3, KLF4, and c-MYC.

In some embodiments, the composition comprises GATA1, SOX1, KLF2, and c-MYC.

In some embodiments, the composition comprises GATA2, SOX1, KLF2, and c-MYC.

In some embodiments, the composition comprises GATA3, SOX1, KLF2, and c-MYC.

In some embodiments, the composition comprises GATA4, SOX1, KLF2, and c-MYC.

In some embodiments, the composition comprises GATA6, SOX1, KLF2, and c-MYC.

In some embodiments, the composition comprises NKX3.1, SOX1, KLF2, and c-MYC.

In some embodiments, the composition comprises OCT4, SOX1, KLF2, and c-MYC.

In some embodiments, the composition comprises GATA3, SOX2, KLF2, and c-MYC.

In some embodiments, the composition comprises GATA4, SOX2, KLF2, and c-MYC.

In some embodiments, the composition comprises GATA6, SOX2, KLF2, and c-MYC.

In some embodiments, the composition comprises GATA1, SOX3, KLF2, and c-MYC.

In some embodiments, the composition comprises GATA2, SOX3, KLF2, and c-MYC.

In some embodiments, the composition comprises GATA6, SOX3, KLF2, and c-MYC.

In some embodiments, the composition comprises OCT4, SOX3, KLF2, and c-MYC.

In some embodiments, the composition comprises KLF4, OCT4, GLIS1, and c-MYC.

In some embodiments, the composition comprises KLF2, OCT4, GLIS1, and c-MYC.

In some embodiments, the factor is a polypeptide, a nucleic acid encoding the same, or a mixture thereof.

For example, the SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC is a protein, a fusion protein bound to a transmembrane domain thereof, or a nucleic acid encoding the same, e.g., in the form of DNA or RNA.

In some embodiments, the factor is provided in the form of an expression vector, and the expression vector may be a plasmid vector, an add-on vector, a transposon, or a virus-derived vector such as an adenovirus vector, an adeno-associated virus vector (AAV), a lentivirus vector, a Sendai virus vector, a cytomegalovirus vector, or a cowpox virus vector.

In some embodiments, the factors are present on one or more expression vectors.

In some embodiments, the factors are present on a same expression vector.

In some embodiments, the factor is in the form of mRNA.

In some embodiments, the factors are linked in tandem on the same mRNA molecule, or each of the factors is on a separate mRNA molecule, wherein the factors are in the form of a complex of multiple mRNA molecules.

In some embodiments, the factor is a synthetic mRNA, and the synthetic mRNA encodes a wild-type form, a mutant that retains activity thereof, or a genetically engineered form of any one selected from the group consisting of SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, and c-MYC.

In some embodiments, the composition further comprises a pharmaceutically acceptable carrier or adjuvant.

In some embodiments, the subject is a mammal, including, but not limited to, human, mouse, rat, bovine, ovine, equine, canine, feline, porcine, or monkey.

In some embodiments, the subject is chicken, duck, goose, or bird.

In some embodiments, the cell, tissue, or organ of a subject is derived from, but not limited to, eye, ear, nose, mouth, including gum and root; bone, lung, breast, pancreas, stomach, or esophagus; muscle, including cardiac muscle; liver, or blood vessel; skin, including hair; heart, brain, neural tissue, kidney, testis, prostate, penis, cloaca, fin, ovary, or intestine.

In some embodiments, the composition for rejuvenating a cell, tissue, or organ of a subject can restore epigenetic information in the cell, tissue, or organ of the subject.

In some embodiments, the composition for rejuvenating a cell, tissue, or organ of a subject can restore loss of epigenetic information due to aging, injury, or disease in the cell, tissue, or organ of the subject.

In some embodiments, a promoter may be used to regulate the expression of a factor to reduce and reverse epigenetic marks associated with aging, increase epigenetic marks associated with cellular rejuvenation, reduce the expression of aging-associated protein, increase the expression of protein associated with cellular rejuvenation, restore the balance between euchromatin and heterochromatin, prevent the loss of cellular identity, restore cellular identity, and reverse aging-associated DNA methylation change, thereby rejuvenating the cell.

In some embodiments, the SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC is a human protein, or a non-human protein, e.g., a protein from a mammal (e.g., mouse, rat, bovine, ovine, equine, canine, feline, porcine, monkey, chicken, duck, goose, and bird), or a protein having at least 80% identity to the amino acid sequence of the corresponding human or non-human protein and maintaining activity.

In some embodiments, the SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, c-MYC, or any combination thereof is a nucleic acid encoding a human protein or a non-human protein, e.g., a nucleic acid encoding a protein from a mammal (e.g., mouse, rat, bovine, ovine, equine, canine, feline, porcine, monkey, chicken, duck, goose, and bird) or a nucleic acid having at least 80% identity to the nucleic acid sequence of the corresponding nucleic acid encoding a human protein or a non-human protein, and maintaining activity.

In some embodiments, the method induces cell reprogramming, reverses aging, improves tissue function, improves organ function, promotes tissue repair, promotes tissue survival, promotes tissue regeneration, promotes tissue growth, promotes angiogenesis, reduces scar formation, attenuates the external manifestations of aging including alopecia areata, thinning of hair, graying of hair, sagging of the skin, and wrinkling of the skin, promotes organ regeneration, promotes organ survival, treatment of diseases or any combination thereof.

In some embodiments, the method reverses the aging of a cell, tissue, organ, or a subject, thereby rejuvenating the cell, tissue, or organ.

In some embodiments, the method may promote neuronal regeneration in a subject.

In some embodiments, the composition may protect neurons in a subject from damage or degeneration.

In some embodiments, the method may reverse the epigenetic age of skin fibroblasts in a subject.

In some embodiments, the method may reverse senescence of a skin fibroblast in a subject.

In some embodiments, the method may reverse senescence of a chondrocyte in a subject.

In some embodiments, the method may reverse senescence of a muscle stem cell in a subject.

In some embodiments, the method may promote improvement of osteoarthritis symptom in a subject.

In some embodiments, the method may promote improvement of glaucoma symptom in a subject.

In some embodiments, the composition may promote the regeneration of optic nerve.

In some embodiments, the composition may promote the axonal regeneration of optic nerve.

In some embodiments, the composition may inhibit the apoptosis of optic nerve cells (e.g., retinal ganglion cells) in the optic nerve injury.

In some embodiments, the method does not induce teratoma formation, or does not induce tumor growth or tumor formation.

In some embodiments, the method does not induce complete reprogramming.

In some embodiments, the method does not reprogram a cell to a pluripotent state, or the method does not reprogram a cell to an "induced multipotent stem cell" or "iPSC".

In a third aspect, the present invention provides use of the composition of the first aspect in the preparation of a medicament for rejuvenating a cell, tissue, or organ of a subject.

In some embodiments, the rejuvenating a cell, tissue, or organ of a subject comprises restoring lost transcriptional profile or epigenetic information in the at least one cell, tissue, or organ due to aging, injury, disease, or any combination thereof.

In some embodiments, the rejuvenating a cell, tissue, or organ of a subject comprises restoring the function of the cell, increasing the potential of the cell, enhancing the viability of the cell, or increasing the replicative capacity or lifespan of the cell, or a combination thereof.

In some embodiments, the rejuvenating a cell, tissue, or organ of a subject comprises promoting axonal regeneration of a neuronal cell in a subject; reversing the epigenetic age of a skin fibroblast in a subject; reversing senescence of a skin fibroblast in a subject; reversing senescence of a chondrocyte in a subject; reversing senescence of a muscle stem cell in a subject; promoting improvement of osteoarthritis symptoms in a subject; or promoting improvement of ocular disease symptoms in a subject.

In a fourth aspect, the present invention provides a kit comprising an inducing factor for rejuvenating somatic cells in a subject, comprising an inducing factor. In some embodiments of the present invention, the kit further comprises a reagent and a vector for reprogramming cells. The vector may be, for example, a vector of a lentiviral system. The kit of the present invention may further comprise a culture medium and an instruction manual.

In some embodiments of the present invention, the inducing factor used comprises: any one factor selected from the group consisting of SOX1, SOX2, SOX3, RCOR2, GMNN, and GLIS1; any one factor selected from the group consisting of GATA1, GATA2, GATA3, GATA4, GATA6, FOXA2, SOX7, SOX17, NKX3.1, NR5A2, C/ EBPα, OCT4, and PAX1; and, factor Klf4 or Klf2. In some embodiments of the present invention, the kit of the present invention further comprises c-MYC.

In some embodiments, the subject suffers from, is suspected of suffering from, or is at risk of suffering from:
a disease, condition, or symptom associated with impaired cell, tissue, or organ viability, or with aging;
the disease, condition, or symptom associated with impaired cell, tissue, or organ viability, or with aging is a neurodegenerative disease, a cardiovascular disease, a metabolic disease, a musculoskeletal disease, an inflammatory disease, a symptom or condition associated with the skin, an ocular disease, a fibrotic disease, or a disease associated with the reproductive system;
the neurodegenerative disease is stroke, Huntington's chorea, dementia, Alzheimer's disease, or Parkinson's disease;
the cardiovascular disease is hypertension, coronary arrhythmia, heart disease, hypotension, heart failure, angina pectoris, arteriosclerosis, myocardial necrosis, myocarditis, congestive heart failure, atrioventricular block, atherosclerosis, or myocardial infarction;
the metabolic disease is type I diabetes, type II diabetes, hyperglycemia, hyperinsulinemia, insulin resistance, obesity, or gout;
the musculoskeletal disease is muscular dystrophy, myasthenia, myasthenia gravis, osteoporosis, osteoarthritis, osteochondrosis, osteochondritis, osteonecrosis, or osteopenia;
the inflammatory disease is systemic lupus erythematosus, rheumatic polymyalgia, gouty arthritis, degenerative arthritis, rheumatoid arthritis, inflammatory arthritis, Hashimoto's thyroiditis, inflammatory bowel disease, hepatitis, pneumonia, respiratory inflammation, or encephalitis;
the symptom or condition associated with the skin is wrinkles, age spots, seborrheic keratosis, tinea manuum and pedis, urticaria, neurodermatitis, or pigmented nevi;
the ocular disease is macular degeneration, retinal degeneration, retinal detachment, diabetic retinopathy, glaucoma, optic atrophy, floaters, cataract, non-arteritic anterior ischemic optic neuropathy, chorioretinitis, retinitis pigmentosa, or optic nerve injury;
the fibrotic disease is pulmonary fibrosis, hepatic fibrosis, myocardial fibrosis, renal interstitial fibrosis, myelofibrosis, pleural fibrosis, or intestinal wall fibrosis; and
the disease associated with the reproductive system is erectile dysfunction or premature ovarian failure.

The present invention screens for novel combination of senescence reprogramming factors that will have broad application in the treatment and/or improvement of many diseases and conditions associated with aging or impaired cell viability.

It is unexpectedly discovered in the present invention that a three-factor combination of any one factor selected from the group consisting of SOX1, SOX2, SOX3, RCOR2, GMNN, and GLIS1, any one factor selected from the group consisting of GATA1, GATA2, GATA3, GATA4, GATA6, FOXA2, SOX7, SOX17, NKX3.1, NR5A2, C/EBPα, OCT4, and PAX1, and factor Klf4 or Klf2, or a combination of any one factor selected from of the group consisting of SOX1, SOX2, SOX3, RCOR2, GMNN, and GLIS1, any one factor selected from of the group consisting of GATA1, GATA2, GATA3, GATA4, GATA6, FOXA2, SOX7, SOX17, NKX3.1, NR5A2, C/EBPα, OCT4, and PAX1, factor Klf4 or Klf2, and factor c-MYC can achieve reversal of senescent cell orthe rejuvenation of cells.

The "factor" in the present invention refers to a biologically active protein, or a nucleic acid encoding the same, such as DNA, RNA, or mixtures thereof, which acts on a cell to alter transcription and which, when expressed, transforms senescent cells into young cells. In some embodiments, the factor may be non-integrative, i.e., is provided to the recipient somatic cell in a form that does not result in integration of the exogenous DNA into the recipient cell's genome.

The factor in the present invention may be a transcription factor including, but not limited to, SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, and c-MYC.

The reprogramming in the present invention is incomplete reprogramming and/or partial reprogramming and/or transient reprogramming. It should be understood that "incomplete" and/or "partial" and/or "transient" reprogramming mentioned herein is compared to a cell with high level of capacity (e.g., ES cell or iPS cell). In another embodiment, this reprogramming of somatic cell is incomplete and/or partial and/or transient reprogramming compared to iPS cell. The rejuvenated cell, tissue or organ does not express at least one stem cell marker (e.g., NANOG) after administration of the composition. Molecular markers associated with partial reprogramming to restore youthfulness include: the epigenetic age, the transcriptome feature, the number of γ -H2AX foci, the concentration of reactive oxygen species, the enrichment of histone marker (e.g., H3K9me3 and H4K20me3), the collagen level, the level of vimentin and E-cadherin, the aging-associated β-galactosidase activity, the cellular proliferation rate, and/or the karyotypic feature.

As used herein, "incomplete" and/or "partially" and/or "transicent" reprogramming comprises a reprogrammed somatic cell that retains and/or includes the phenotype of the unreprogrammed somatic cell. The retention and/or inclusion of such phenotype of the unreprogrammed somatic cell comprises the retention of the expression of surface marker indicative of the cellular lineage or identity of the somatic cell. In addition, such retention and/or inclusion may also comprise the epigenetic marker of the cellular lineage or identity of the unreprogrammed somatic cells being retained and/or included by the reprogrammed somatic cell. Thus, in one embodiment, the reprogrammed somatic cell comprises the phenotype of the unreprogrammed somatic cell. In another embodiment, the reprogrammed somatic cell retains and/or includes the phenotype of the unreprogrammed somatic cell of the tissue from which the reprogrammed somatic cell is obtained. In another embodiment, the reprogrammed somatic cell retains and/or includes the phenotype and/or epigenetic marker indicative of the cellular lineage or identity of the somatic cell.

Epigenetic alteration is one of the most important features of aging, and is manifested primarily as changes in the methylation status of the whole genome with increasing age at the chromatin level.

Cell age of different tissue types is measured by using epigenetic age for predicting aging and validating effective anti-aging interventions. In addition to this, the perturbation of the autophagy-lysosome system in senescent cells can lead to an increase in the lysosomal content of the cells, thereby resulting in the accumulation of auxiliary lysosomal senescence-associated β-galactosidase in senescent cell. Therefore, the detection of β-galactosidase content in the cells by histochemistry is also a common method for determining the senescent status of cells.

The cells herein comprise any type of cell that constitutes an organism. Thus, the somatic cell may include a cell from, for example, eye, ear, nose, mouth including gum and root, bone, cartilage, lung, breast, udder, pancreas, stomach, esophagus, muscle including cardiac muscle, liver, blood vessel, skin including hair, heart, brain, neural tissue, kidney, testis, prostate, penis, cloaca, fin, ovary, or intestine. In one embodiment of the present invention, the somatic cell is a cell from a connective tissue, such as a skin fibroblast. In one embodiment, the somatic cell is a chondrocyte. In one embodiment, the somatic cell is a muscle stem cell. In another embodiment, the somatic cell is a neural cell, e.g., a cell from the central and/or peripheral nervous system. Thus, in one embodiment, the cell is an optic neuron. In another embodiment, the cell is a sensory neuron. In an alternative embodiment, the cell is a motor neuron. In another embodiment, the cell is an interneuron. In one embodiment, the somatic cell is derived from an animal. In another embodiment, the somatic cell is derived from a mammal. In another embodiment, the mammal is a human. Thus, in a specific embodiment, the somatic cell is derived from a human and is a human somatic cell. In an alternative embodiment, the mammal is a mouse, and the somatic cell is a mouse somatic cell.

In another embodiment, the reprogrammed somatic cell comprises such a molecular marker, e.g., an epigenetic marker, which is indicative of a younger or earlier epigenetic age than the non-reprogrammed somatic cell or the somatic cell from the tissue or organism from which the reprogrammed somatic cell was obtained. In other embodiments, the reprogrammed somatic cell generated according to the method defined herein comprises a phenotype and/or molecular marker similar to the phenotype and/or molecular marker of the unreprogrammed somatic cell, such as epigenetic marker.

In some embodiments, the tissue or organ as defined herein is selected from: eye, ear, nose, mouth including gum and root, bone, cartilage, lung, breast, udder, pancreas, stomach, esophagus, muscle including cardiac muscle, liver, blood vessel, skin including hair, heart, brain, nervous tissue, kidney, testis, prostate, penis, cloaca, fin, ovary, or intestine.

In some embodiments, the composition induces cell reprogramming, reverses aging, improves tissue function, improves organ function, promotes tissue repair, promotes tissue survival, promotes tissue regeneration, promotes tissue growth, promotes angiogenesis, reduces scar formation, attenuates the external manifestations of aging including alopecia areata, thinning of hair, graying of hair, sagging of the skin, and wrinkling of the skin, promotes organ regeneration, promotes organ survival, treatment of disease or any combination thereof.

In some embodiments, the method does not induce teratoma formation or does not induce tumor growth or tumor formation.

As used herein, the inducing factors, used in its broadest sense, include a nucleic acid that produces a reprogrammed cell, a chemical agent, an engineered protein, and any recombinant virus.

### BRIEF DESCRIPTION OF THE DRAWINGS

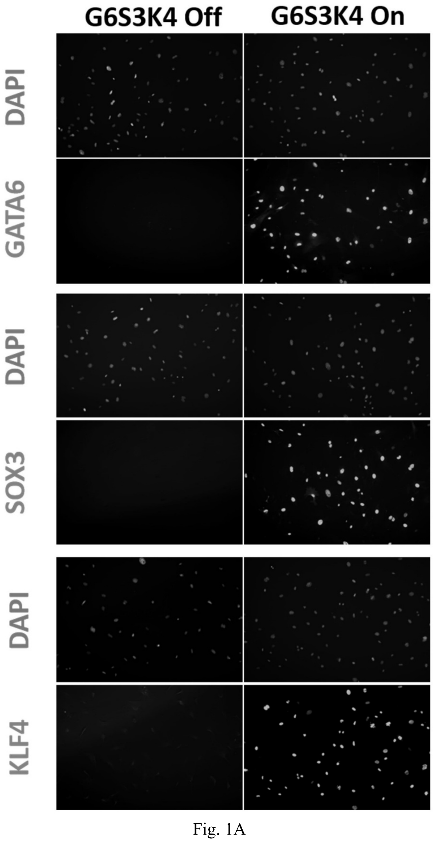

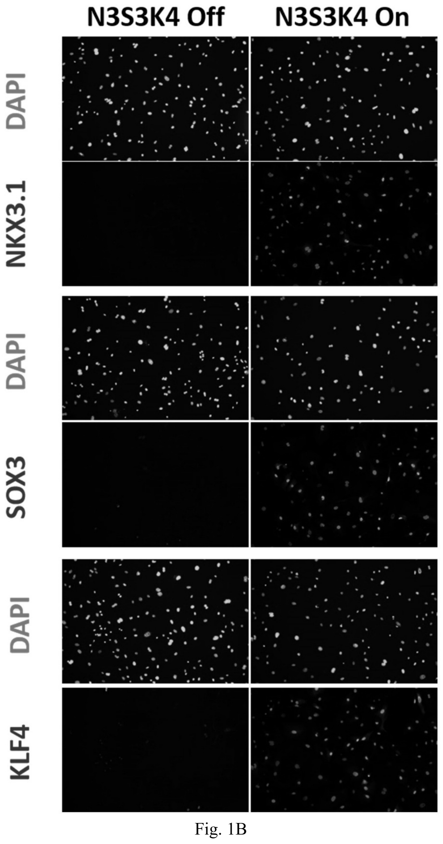

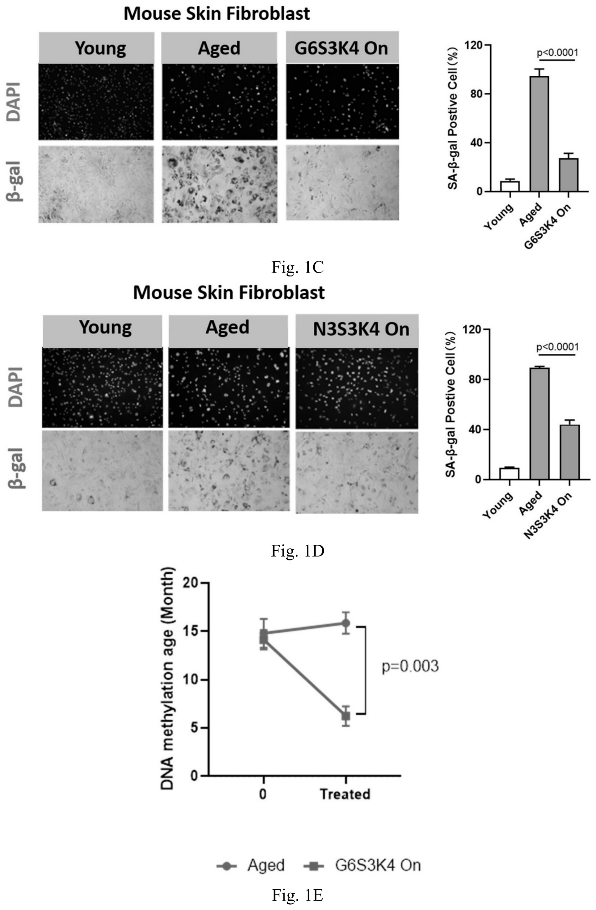

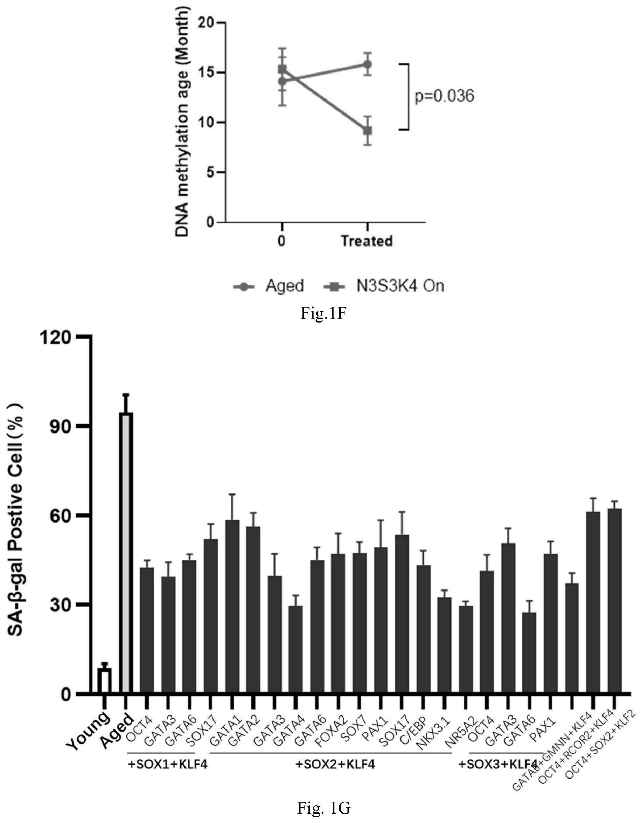

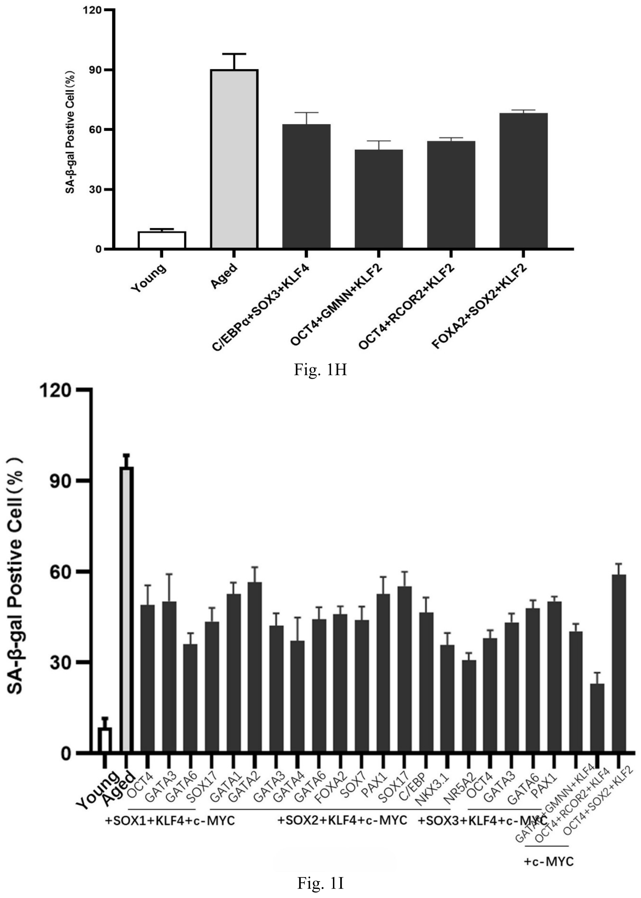

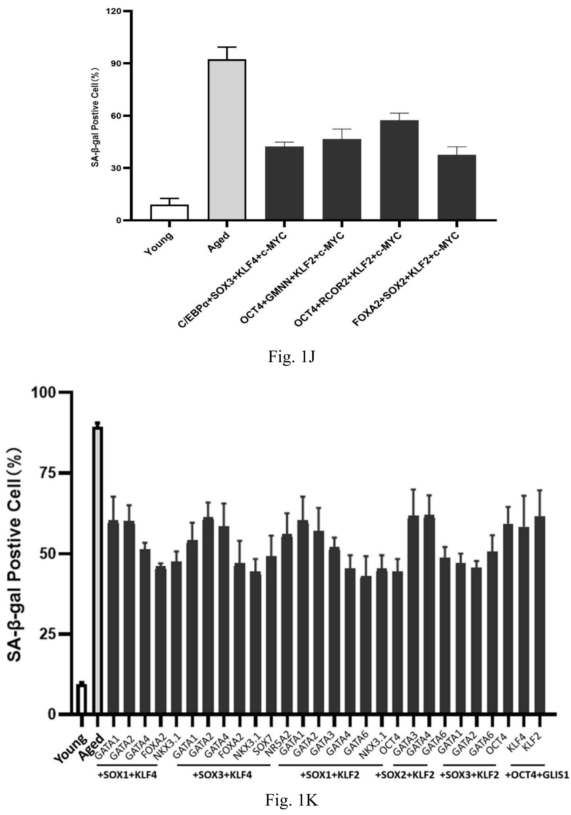

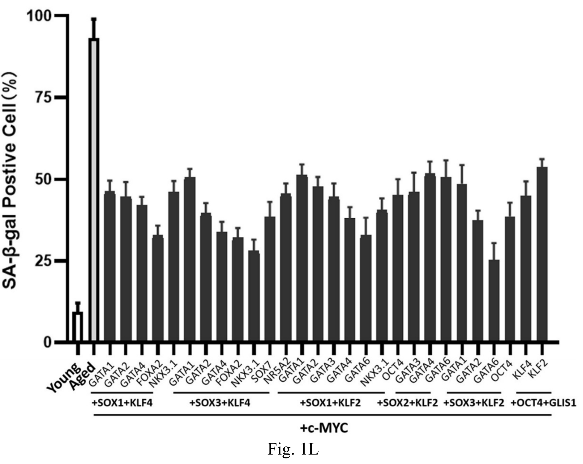

Figure 1A to Figure 1L illustrate that the reprogramming restores the aging physiological properties in skin fibroblasts of an aged mouse to a more youthful state. Figure 1A and Figure 1B illustrate the expression of relevant genes in cells after lentiviral transfection. Figure 1C and Figure 1D illustrate representative graphs of the changes in β-galactosidase activity before and after treatment: 5 days of the reprogramming utilizing the combinations of GATA6+SOX3+Klf4 and NKX3.1+SOX3+KLF4. Figure 1E and Figure 1F illustrate DNA methylation age changes (i.e., the changes in epigenetic age) in skin fibroblasts of a mouse after reprogramming using the compositions of GATA6+SOX3+Klf4 and NKX3.1+SOX3+KLF4, respectively. Figure 1G to Figure 1L illustrate the effects of the different combinations of GATA1, GATA2, GATA3, GATA4, GATA6, FOXA2, SOX7, SOX17, NKX3.1, NR5A2, C/EBPα, OCT4, PAX1, SOX1, SOX2, SOX3, RCOR2, GMNN, Klf2, Klf4, GLIS1, and c-MYC genes on β-galactosidase activity. All bar graphs were generated after pooling data from biological and technical replicates of all individual cells for ease of viewing. The shown significance levels allowed a wide margin for pairwise comparisons of differences between patients.

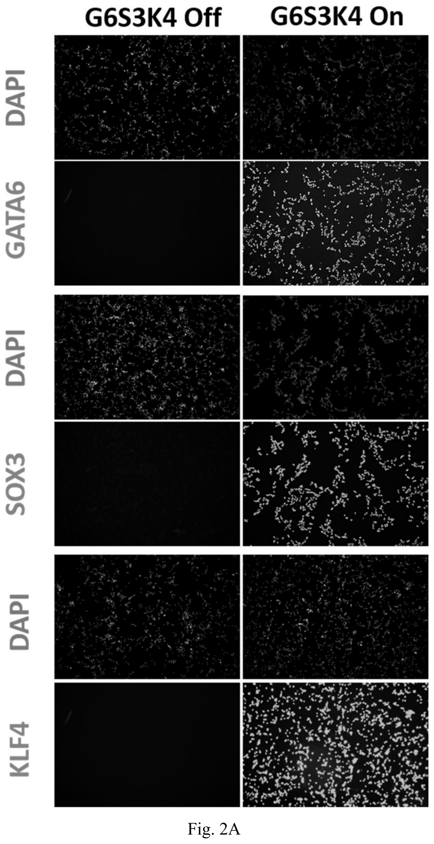

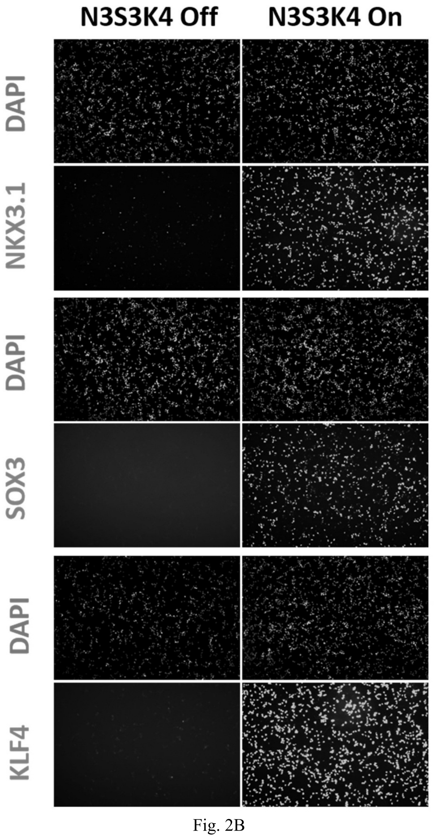

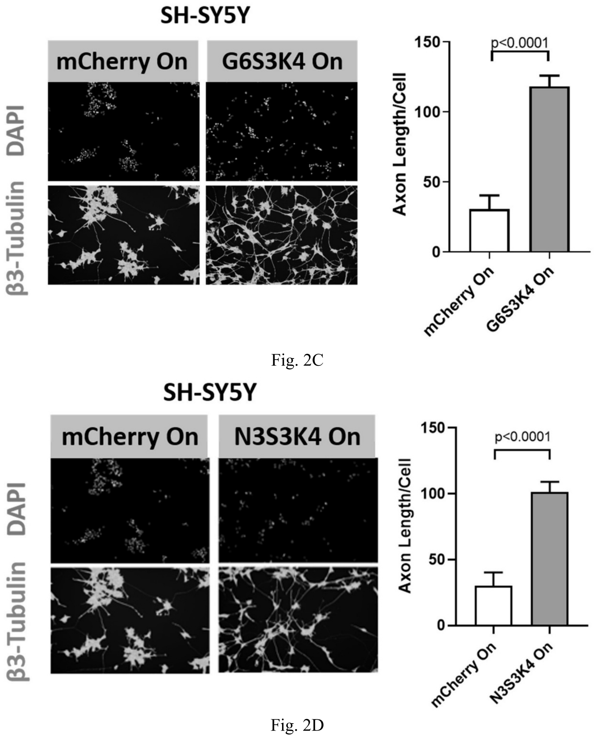

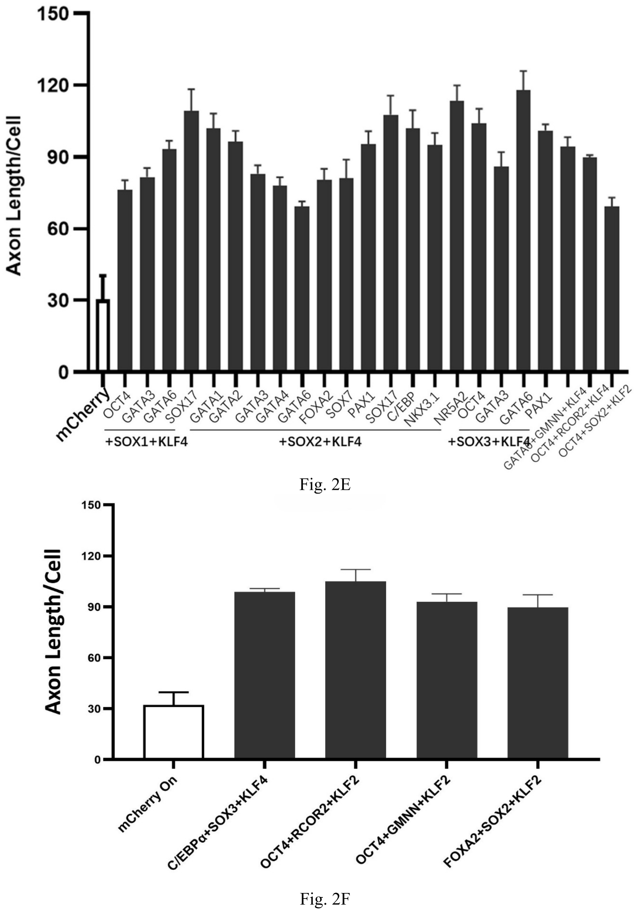

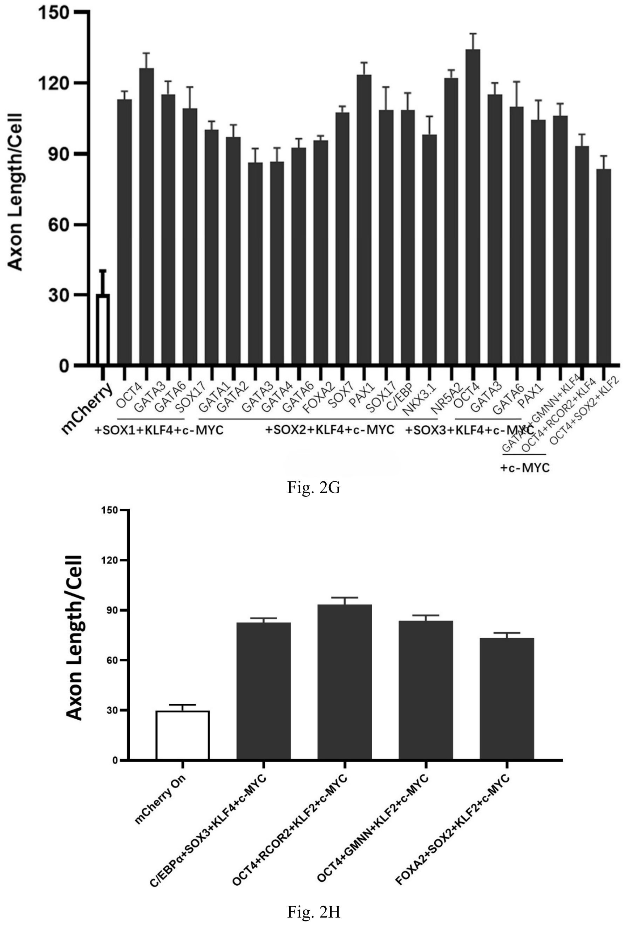

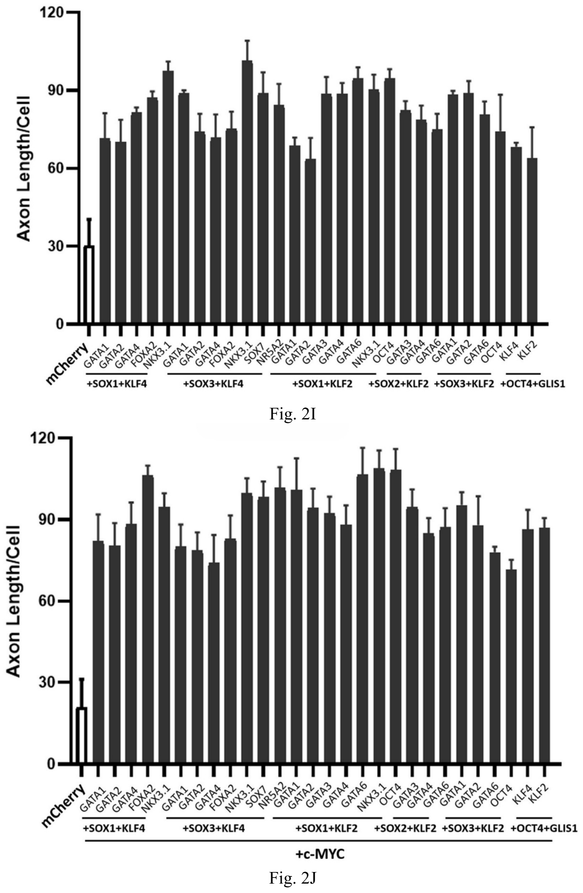

Figure 2A to Figure 2J illustrate that the reprogramming promotes neuronal survival and axonal regeneration in human SH-SY5Y neuronal cells after vincristine (VCR)-induced damage. Figure 2A and Figure 2B illustrate the expression of relevant genes in cells after lentiviral transfection. Figure 2C and Figure 2D illustrate representative graphs of changes in synapse length before and after treatment: 6 days of the reprogramming utilizing the combinations of GATA6+SOX3+Klf4 and NKX3.1+SOX3+KLF4. Figure 2E to Figure 2J illustrate the effect of the different cmbinations of GATA1, GATA2, GATA3, GATA4, GATA6, FOXA2, SOX7, SOX17, NKX3.1, NR5A2, C/EBPα, OCT4, PAX1, SOX1, SOX2, SOX3, RCOR2, GMNN, Klf2, Klf4, GLIS1, and c-MYC genes on synapse length. All bar graphs were generated after pooling data from biological and technical replicates of all individual cells for ease of viewing. The shown significance levels allowed a wide margin for pairwise comparisons of differences between patients.

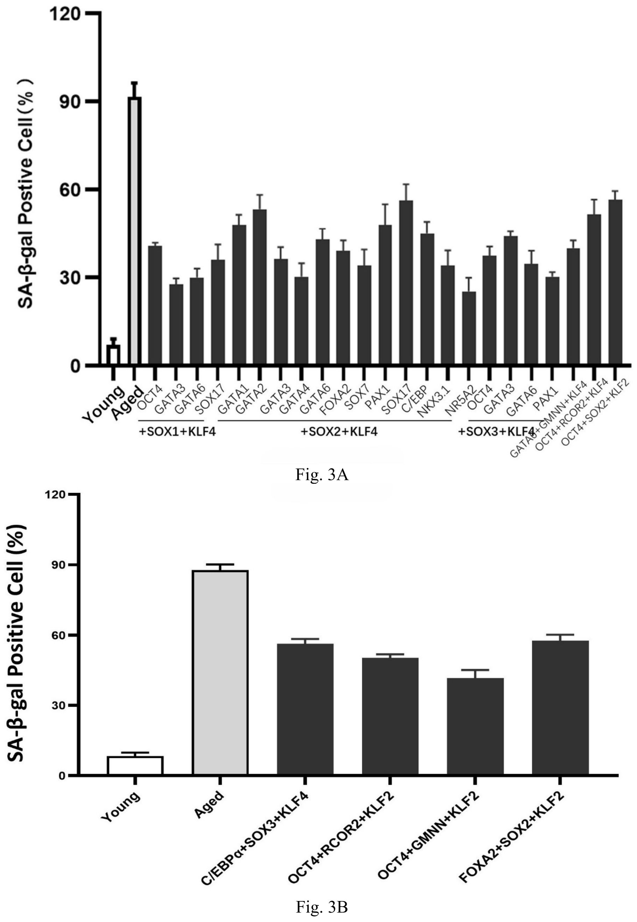

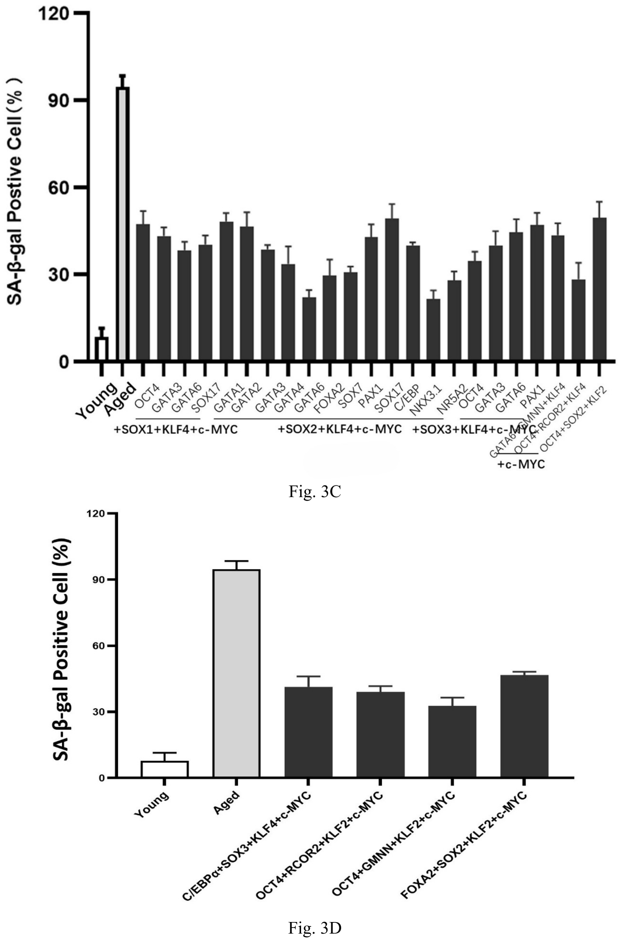

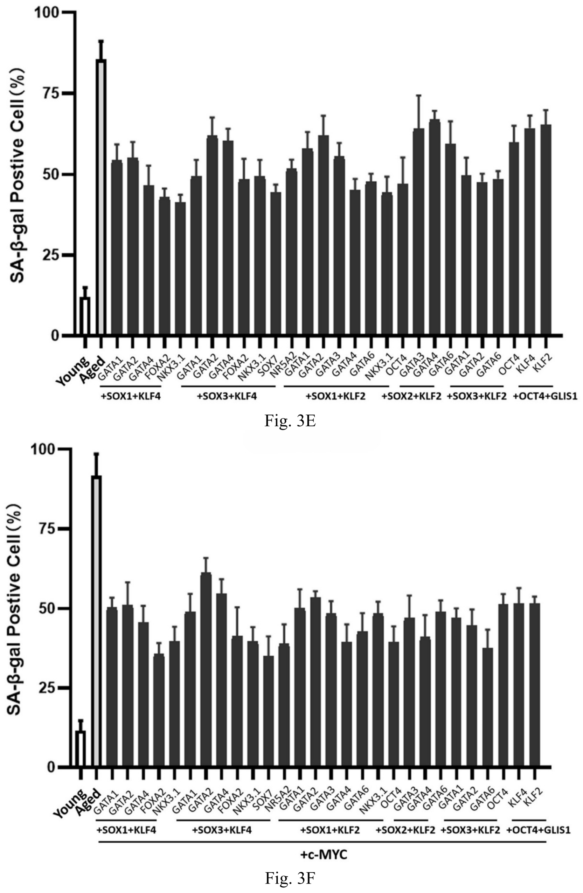

Figure 3A to Figure 3F illustrate that the reprogramming restores the aging physiological properties in chondrocytes to a more youthful state: β-galactosidase activity in chondrocytes was assayed after 5 days of reprogramming using the different combinations of GATA1, GATA2, GATA3, GATA4, GATA6, FOXA2, SOX7, SOX17, NKX3.1, NR5A2, C/EBPα, OCT4, PAX1, SOX1, SOX2, SOX3, RCOR2, GMNN, Klf4, Klf2, GLIS1, and c-MYC genes. All bar graphs were generated after pooling data from biological and technical replicates of all individual cells for ease of viewing. The shown significance levels allowed a wide margin for pairwise comparisons of differences between patients.

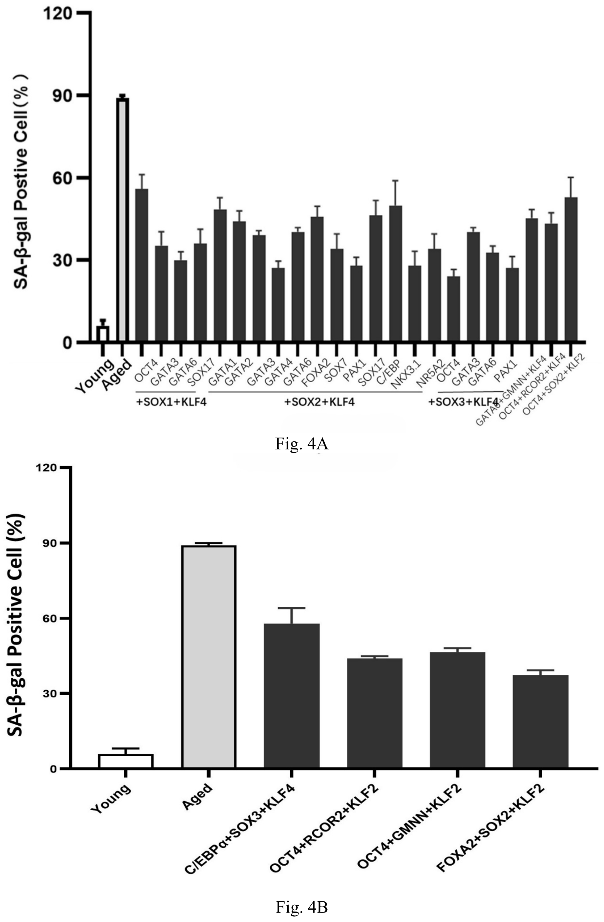

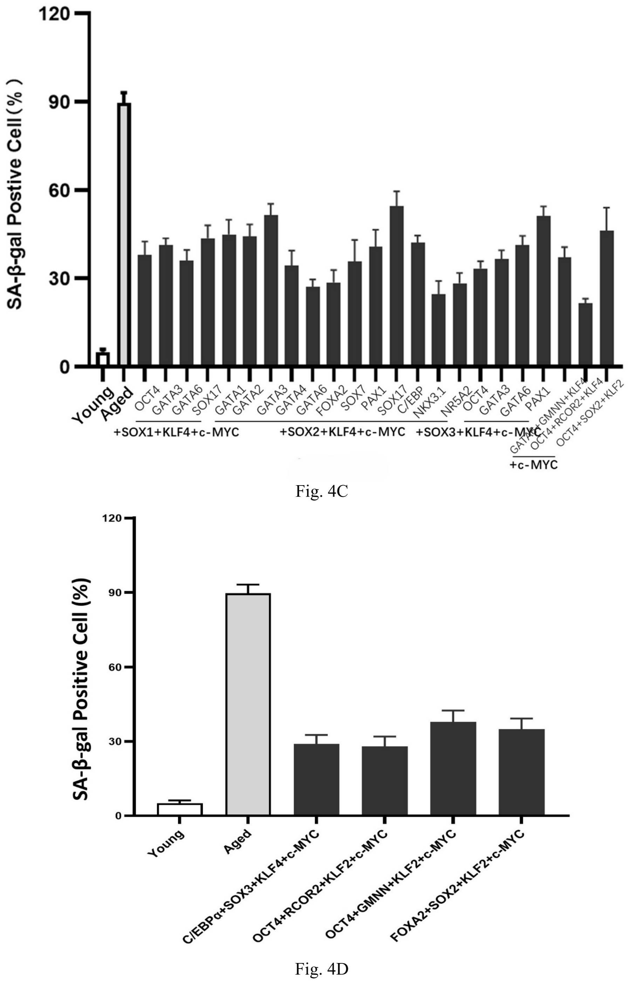

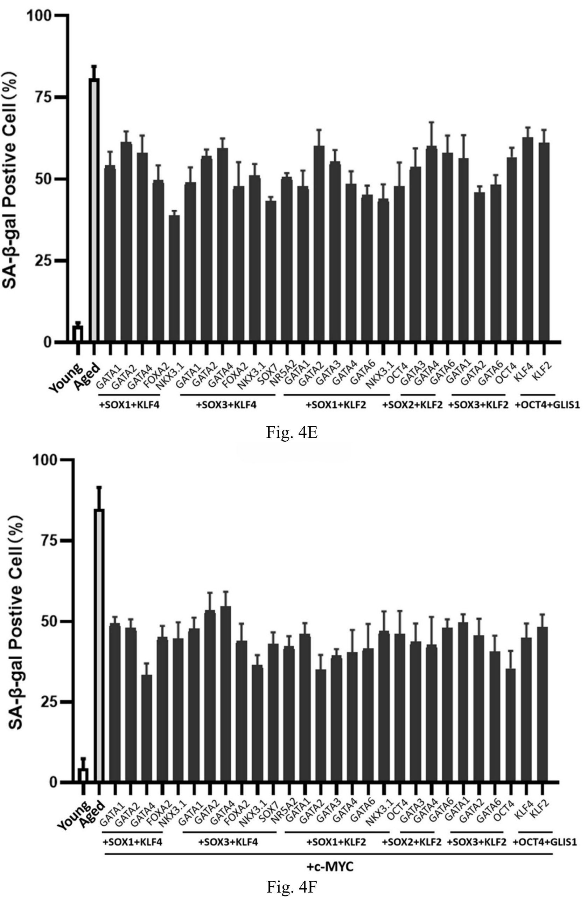

Figure 4A to Figure 4F illustrate that the reprogramming restores the aging physiological properties in muscle stem cells of an aged mouse to a more youthful state: β-galactosidase activity in muscle stem cells of the mouse was assayed after 5 days of reprogramming using the different combinations of GATA1, GATA2, GATA3, GATA4, GATA6, FOXA2, SOX7, SOX17, NKX3.1, NR5A2, C/EBPα, OCT4, PAX1, SOX1, SOX2, SOX3, RCOR2, GMNN, Klf4, Klf2, GLIS1, and c-MYC genes. All bar graphs were generated after pooling data from biological and technical replicates of all individual cells for ease of viewing. The shown significance levels allowed a wide margin for pairwise comparisons of differences between patients.

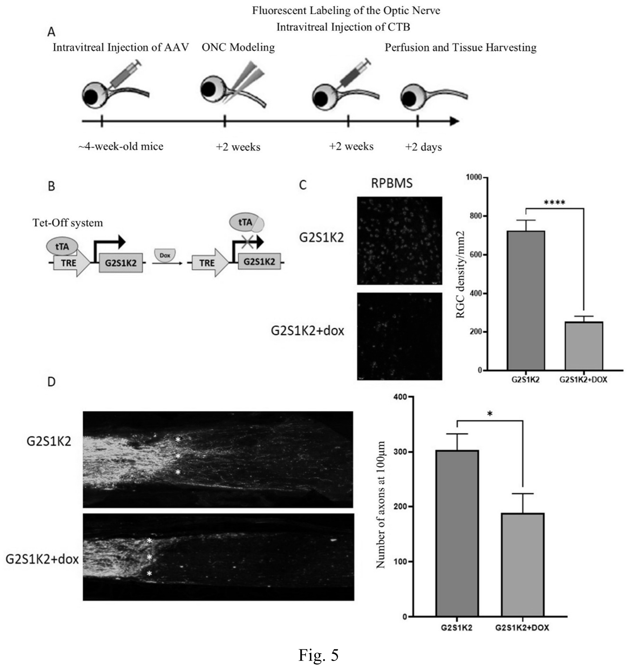

Figure 5 illustrates the effect of reprogramming to promote optic nerve regeneration. Figure 5A illustrates a schematic diagram of the ONC pharmacodynamic assay process; Figure 5B illustrates the AAV virus expression pattern; Figure 5C illustrates the effect of G2S1K2 on RGC apoptosis; and Figure 5D illustrates the effect of G2S1K2 on optic nerve regeneration.

### DETAILED DESCRIPTION OF THE EMBODIMENTS

The the present invention is further illustrated through examples below. One of ordinary skills in the art will appreciate that the invention is not limited to the described examples, and one of ordinary skills in the art can modify the examples based on the teachings of the specification. These modifications are likewise encompassed within the scope of the present invention as defined herein by the appended claims.

The experimental methods in the following examples are conventional unless otherwise specified.

### 1. Mouse strains

ICR mice were purchased from Charles River.

### 2. Viral transfection

The nucleic acid sequences encoding the factors of interest were inserted into FUW-TetON lentiviral vectors respectively and transduced into 293T cells to package the lentiviruses. The supernatant of each virus was collected after 72 h and aliquoted and cryopreserved at -80°C after filtered through a 0.22 µm membrane. Lentiviral combinations of the factors of interest were obtained by mixing the supernatants containing lentiviruses expressing each of the factors of interest respectively in the presence of polybrene (10 µg/ml), and these lentiviral combinatiions were simultaneously used to infect mouse skin fibroblasts, mouse chondrocytes, and mouse muscle stem cells. The transfection of SH-SY5Y neuronal cellst was performed under polybrene-free conditions. After 12 hours of transfection, the medium containing 1 µg/ml doxycycline was changed for the cells to induce expression for at least 3 days.

The inducing factors used in the examples include:
GATA1 (AAV38577.1), GATA2 (NCBI Reference Sequence: NP_001139133.1), GATA3 (AAX36808.1), GATA4 (NCBI Reference Sequence: NP_001295022.1), GATA6 (NCBI Reference Sequence: NP_005248.2), SOX1 (NCBI Reference Sequence: NP_005977.2), SOX2 (CAA83435.1), SOX3 (NCBI Reference Sequence: NP_005625.2), SOX7 (AAP36695.1), SOX17 (BAB83867.1), OCT4 (PDB: 6T90_K), FOXA2 (NCBI Reference Sequence: NP_068556.2), PAX1 (NCBI Reference Sequence: NP_006183.2), C/EBPα (NCBI Reference Sequence: NP_004355.2), NKX3.1 (NCBI Reference Sequence: NP_006158.2), NR5A2 (NCBI Reference Sequence: NP_995582.1), KLF2 (NCBI Reference Sequence: NP_057354.1), KLF4 (NCBI Reference Sequence: NP_004226.3), GMNN (NCBI Reference Sequence: NP_001238918.1), RCOR2 (NCBI Reference Sequence: XP_047282784.1), c-MYC(BAA01374.2) and GLIS1 (NCBI Reference Sequence: NP_001354413.1).

### 3. Extraction of skin fibroblasts from aged mice

24-month-old aged mice were selected, and after euthanasia of the mice, the shaved dorsal skin was washed with iodophor (povidone-iodine) and rinsed with cold PBS (1x) and then excised. The fibroblasts of the mice were separatedwith trypsin and collagenase. The fibroblasts were resuspended and cultured with DMEM GlutaMax medium.

8-week-old mice were selected as young mice.

### 4. Differentiation of SH-SY5Y cells into neurons

1 day after plating, the cells began differention in EMEM/F12 medium (1:1) containing 5% FBS and 10 µM all-trans retinoic acid (RA, MCE) (differentiation medium 1) for 5 days (the medium was changed every three days).Then the medium was changed to serum-free neurobasal/B27 plus medium (Gibco) containing 1× Glutamax (Gibco) and 50 ng/ml BDNF (MCE) (differentiation medium 2) for additional 5 days of maturation.

### 5. Senescence induction of chondrocyte

Human chondrocyte cell line C28/I2 was plated. 1 day after plating, 20 µM of etoposide was added to the cells for senescence induction. After 24 hours of induction, the medium was changed to fresh complete medium DMEM (Gibco) (containing 10% of FBS (Gibco) and 1% of P/S (Gibco)) for additional 3 days of culture. Then the composition was added for treatment for 5 days, and the fixation and subsequent treatment can be carried out.

### 6. Extraction of muscle stem cells from aged mice

24-month-old aged mice were selected and executed by cervical dislocation. The muscles were isolated, cut into small pieces, and incubated in DMEM medium (DMEM; ThermoFisher Scientific) containing 0.2% of type I collagenase (Sigma-Aldrich) at 37 °C for 2 hours. Single fibers were collected using a pipette tip and purified twice in DMEM containing 1 g/L of glucose supplemented with 10% of equine serum (HS, ThermoFisher Scientific), 1% penicillin/streptomycin (PS, ThermoFisher Scientific), and 0.5% chicken embryo extract (CEE, ThermoFisher Scientific). Then, single fibers were transferred and the myofiber suspension was passed through a syringe with a 21G needle and filtered through a 40 µm filter. The obtained stem cells were plated directly on a culture dish coated with Matrigel Growth Factor (GFR) Basement Membrane Matrix (Corning) or a culture dish containing a coverslip coated with Matrigel GFR Basement Membrane Matrix. Cells were expanded in DMEM containing 1 g/L of glucose supplemented with 10% of HS, 20% of FBS, 1% of PS, and 0.5% of CEE, and cultured at 37°C with 5% CO₂. The medium was changed every 2 days.

### 7. Detection of β-galactosidase activity

The treated cells were fixed by adding 4% of paraformaldehyde for 15 min and then stained *in situ* using β-galactosidase in situ staining kit (Beyotime). After the staining was completed, DAPI was further added for nuclear staining in order to count the total number of cells. After that, the cells were observed under a fluorescence microscope and the positive cell rate was calculated (positive cell rate = number of stained positive cells/total number of cells × 100%).

### 8. Detection of epigenetic age

The genomic DNA was extracted and purified from the samples using a DNA extraction kit (Qiagen), and the methylation sites in the target regions of the genes were sequenced. The interpreted age of the cells was calculated from the sequencing results of the target CpG sites.

### 9. Neural synapse regeneration assay

Mature neurons differentiated from SH-SY5Y cells were transduced with lentiviral vectors. Cultures were continued for at least 3 days after transduction, and 200 nM of vincristine (VCR, MCE) was added to the cells for 24 hours to induce neural synapse degeneration. After vincristine treatment, the neurons were washed twice in PBS, and then fresh differentiation medium 2 was added back to the plate for about 2 weeks of continued culture to track the regeneration of neuronal synapses.

### 10. Immunofluorescence and imaging

After fixation with 4% of paraformaldehyde for 30 min at room temperature, the cells were permeabilized and blocked with 1% of BSA solution containing 0.25% of TritonX-100 for 1 h; followed by incubation with 1% of BSA solution containing an appropriate proportion of the primary antibody and 0.25% of TritonX-100 overnight at 4°C. The cells were washed with 0.1% of BSA, and then incubated with secondary antibody solution at room temperature for 1 h. All secondary antibodies were Alexa-Fluor-conjugated (Molecular Probes) and diluted at 1:1000 in 1% of BSA. After washing again with 0.1% of BSA, the nuclei were restained with PBS containing 5 µg/mL of DAPI for 5 min. After washing with PBS, the optical sections were captured by a fluorescence microscopy. Fluorescence semi-quantitative analysis was performed using ImageJ.

### Example 1. Reversal effect of the reprogramming on cellular senescence

To investigate the possibility of the reprogramming to reverse senescence, we infected skin fibroblasts from aged mice with lentiviral vectors encoding human GATA6, SOX3, NKX3.1, and KLF4 under a Tet-inducible promoter (using the Tet-on system) in different combinations as shown below.

Figure 1A illustrated the expression of GATA6, SOX3, and KLF4 induced in cells under G6S3K4 On condition.

Figure 1B illustrated the expression of NKX3.1, SOX3, and KLF4 induced in cells under G6S3K4 On condition.

After infection, we examined changes in cellular senescence by β-galactosidase activity assay and epigenetic age analysis, respectively, after treating cells with doxycycline for 5 days. β-galactosidase, one of the earliest and most widely used lysosomal-derived senescence markers, can increase lysosomal biosynthesis in senescent cells and is considered to be a marker of cellular senescence specificity as well as a gold indicator for aging detection.

Skin fibroblasts from aged mice were assayed for β-galactosidase content in the cells 5 days after induction of expression with doxycycline. Relative to untreated cells (Aged group), the cells in which the expression of GATA6, SOX3, and Klf4 was induced (G6S3K4 On condition) showed a reduced β-galactosidase activity (Figure 1C), and the cells in which the expression of NKX3.1, SOX3, and KLF4 was induced (G6S3K4 On condition) also showed a reduced β -galactosidase activity (Figure 1D).

Similarly, the results of the epigenetic age analysis also showed that relative to untreated cells (Aged group), the cells in which the expression of GATA6, SOX3, and Klf4 was induced (G6S3K4 On condition) (Figure 1E) showed a significant reversal of methylation age corresponding to reversing the cell age by about 8 months, and the cells in which the expression of NKX3.1, SOX3, and KLF4 was induced (G6S3K4 On condition) (Figure 1F) also showed a significant reversal of methylation age corresponding to reversing the cell age by about 6 months.

Furthermore, lentiviral vectors expressing GATA1, GATA2, GATA3, GATA4, GATA6, FOXA2, SOX7, SOX17, NKX3.1, NR5A2, C/EBPα, OCT4, PAX1, SOX1, SOX2, SOX3, RCOR2, GMNN, KLF2, KLF4, GLIS1, or c-MYC were differently combined to infect the cells respectively, and then the β-galactosidase activity was examined in skin fibroblasts from aged mice. The results showed that the expression of the above gene combinations, such as NR5A2+SOX2+KLF4, FOXA2+SOX1+KLF4, OCT4+RCOR2+Klf4+c-MYC, FOXA2+SOX1+KLF4+c-MYC, etc., were able to significantly reduce the β-galactosidase activity in the cells (Figure 1G to Figure 1L).

### Example 2. Reversal effect of the reprogramming on SH-SY5Y neuronal cell damage

In order to identify whether the expression of human GATA6, human SOX3 and human Klf4 (human G6S3K4), as well as human NKX3.1, human SOX3 and human KLF4 (human N3S3K4) can protect human neuronal cells and regenerate neuronal synapses in vitro, a synapse regeneration assay was used as described below. SH-SY5Y cells which are human neuroblastoma cells and can differentiate into neuronswere transduced under a Tet-inducible promoter (using the Tet-on system) with lentiviral vectors encoding human GATA6, human SOX3, and human Klf4 (tetOn-hG6S3K4), lentiviral vectors encoding human NKX3.1, human SOX3, and human KLF4 (tetOn-h N3S3K4), or lentiviral vector encoding mCherry.

Figure 2A illustrates the expression of human GATA6, human SOX3, and human Klf4 induced in cells under G6S3K4 On condition.

Figure 2B illustrates the expression of human NKX3.1, human SOX3, and human KLF4 induced in cells under N3S3K4 On condition.

3 days after transduction, the cells were treated with vincristine (VCR) for 24 hours to induce neuronal synaptic degeneration. VCR is a chemotherapy drug that destroys microtubules. It is commonly used *in vitro* to determine whether post-damage treatments maintain and/or promote cellular function (e.g., neuronal function). The effects of G6S3K4 treatment on neuronal survival and synaptic regrowth were determined using VCR as described herein. After VCR treatment, the cells were grown in differentiation medium, and the length of neural synapses was determined.

The neuron length of the cells was determined 9 days after the cells were treated with VCR for 24 hours. Relative to the cells in which mCherry expression was induced (mCherry On condition), the cells in which the expression of human GATA6, human SOX3, and human Klf4 was induced (G6S3K4 On condition) showed an increased neuronal survival and synapse length (Figure 2C, left panel). Similarly, the quantification of the length of neural synapses showed that the expression of human GATA6, human SOX3, and human Klf4 increased the length of neural synapses by at least 4-fold compared with mCherry expression (Figure 2C, right panel).

Figure 2D (left panel) illustrates that the cells with the induced expression of human NKX3.1, human SOX3, and human KLF4 (N3S3K4 On condition) showed an increased neuronal survival and axon length. Figure 2D (right panel) illustrates that the quantification of the length of neural synapses showed the expression of human NKX3.1, human SOX3, and human KLF4 increased the length of neural synapses by at least 3-fold compared with mCherry expression.

Furthermore, the GATA1, GATA2, GATA3, GATA4, GATA6, FOXA2, SOX7, SOX17, NKX3.1, NR5A2, C/EBPα, OCT4, PAX1, SOX1, SOX2, SOX3, RCOR2, GMNN, KLF2, KLF4, GLIS1, and c-MYC genes in different combinations were expressed in SH-SY5Y cells, respectively, and the length of neural synapses was examined to test the effect of these combinations on neural synapses regeneration.

The results showed that the expression of the above combinations, such as NR5A2+SOX2+Klf4, OCT4+SOX3+Klf4+c-MYC, FOXA2+SOX3+KLF4, NKX3.1+SOX1+KLF2+c-MYC, etc., significantly promoted the regeneration of neural synapses (Figure 2E to Figure 2J).

These results suggested that the expression of human G6S3K4, N3S3K4 and other combinations protect human neuronal cells from VCR-induced neuronal degeneration.

### Example 3. Reversal effect of the reprogramming on chondrocyte senescence

To determine the reversal effect of new reprogramming combinations on chondrocyte sencescence, a β-galactosidase activity assay was used as described below. The GATA1, GATA2, GATA3, GATA4, GATA6, FOXA2, SOX7, SOX17, NKX3.1, NR5A2, C/EBPα, OCT4, PAX1, SOX1, SOX2, SOX3, RCOR2, GMNN, KLF4, KLF2, GLIS1, and c-MYC genes in different combinations were expressed in induced senescent human chondrocytes, respectively. After 5 days of reprogramming, the β-galactosidase activity in chondrocytes was tested. The results showed that the new gene combinations were able to reduce the β-galactosidase activity in the cells by 60% to 70% relative to the untreated cells (Aged group) (Figure 3A to Figure 3F).

### Example 4. Reversal effect of the reprogramming on muscle stem cell senescence of aged mice

To determine the reversal effect of the new reprogramming combinations on muscle stem cell sencescence of aged mice, a β-galactosidase activity assay was used as described below. The GATA1, GATA2, GATA3, GATA4, GATA6, FOXA2, SOX7, SOX17, NKX3.1, NR5A2, C/EBPα, OCT4 or PAX1, SOX1, SOX2, SOX3, RCOR2, GMNN, KLF4, KLF2, GLIS1, and c-MYC genes in different combinations were expressed in muscle stem cells of aged mice, respectively. After 5 days of reprogramming, the β-galactosidase activity in muscle stem cells of aged mice was tested. The results showed that the new gene combinations were able to reduce the β-galactosidase activity in the cells by about 60% to 70% relative to the untreated cells (Aged group) (Figure 4A to Figure 4F).

### Example 5. Effects of the reprogramming to promote optic nerve regeneration

To further test whether the combination of G2S1K2 (GATA2+ SOX1+ KLF2) can significantly promote optic nerve regeneration and exert senescence reprogramming effects in mice *in vivo,* this Example utilized a mice optic nerve injury model for *in vivo* pharmacodynamic testing. Figure 5A exemplarily depicts a schematic diagram of a process for an *in vivo* pharmacodynamic assay and the detailed procedure is as follows:

### 1. Preparation of AAV-TRE-G2S1K2 and AAV-CAG-tTA viruses:

The coding nucleic acid sequences of GATA2, SOX1, and KLF2 were linked in tandem via the linkers P2A and T2A, respectively (GATA2-P2A-SOX1-T2A-KLF2) and inserted into the AAV-TRE vector (containing a tetracycline-responsive element (TRE3G promoter) and an SV40 element), to form the AAV-TRE-G2S1K2 vector (Figure 5B). AAV-CAG-tTA was purchased from BrainVTA (Wuhan) Co., Ltd.

### 2. Construction of optic nerve crush injury model:

Approximately 4-week-old mice were anesthetized and injuected intravitreally with 1.5 µl of virus solution including 1 µl of AAV-TRE-G2S1K2 and 0.5 µl of AAV-CAG-rTA. After completing the virus injection, the mice were divided into G2S1K2 and G2S1K2+dox groups, 5 mice of each group, in which the mice in G2S1K2+dox group were administrated with drinking water added with 2 mg/ml of doxycycline hydrochloride (Dalian Meilun Biotechnology Co., Ltd., #MB1088-2) to inhibit the expression of G2S1K2 in the virus, and the mice in G2S1K2 group were administrated with normal drinking water.

Two weeks after AAV injection, the mice were deeply anesthetized, and under a dissecting microscope, the crush injury was made by pinching the optic nerve approximately 2 mm from the eyeball for approximately 5 seconds using forceps, and the forceps were withdrawn after pinching. 2 weeks later, the optic nerve was fluorescently labeled by vitreous injection of cholera toxin B subunit (CTB) (thermo, #C34775), and the mice were euthanized for eye sampling 2 days later.

### 3. Eye sampling:

After euthanizing the mice using CO₂, the eyeballs and optic nerves were harvested.

### 4. Retinal flat mounting:

The harvested eyeballs were rinsed with PBS and then fixed in 4% paraformaldehyde solution for 1.5-2 h at room temperature; the anterior cornea, lens and vitreous body were removed from the eyeballs of the fixed mice in PBS and the retina was divided into 4 flaps equally; 0.5% Triton X-100 was added, and the retinas were permeabilized for 30 min on a horizontal shaker; the 0.5% Triton X-100 was aspirated, and a blocking solution (5% of BSA solution containing 0.1% of Triton X-100 prepared in PBS) was added for blocking overnight at 4°C on a horizontal shaker; RBPMS antibody working solution (RBPMS, Abcam, # AB152101, 1:300 dilution ratio) was added, and the retinas were incubated for 2 days in a refrigerator at 4°C; the retinas were washed 3 times with PBS on a shaker; Alexa Fluor^{™} 488 Donkey Anti-Rabbit IgG Antibody working solution (Thermo Fisher, #A-21206, 1:1000 dilution ratio) was added, and the retinas were incubated for 2.5 hours at room temperature on a horizontal shaker; and the retinas were aspirated flat-mounted on a coverslip and sealed with a slide. Photographs were taken at 20× magnification using a confocal microscope. The number of RBPMS-positive cells, i.e., retinal ganglion cell (RGC), in each photograph was counted using Image software. The results were shown in Figure *5C**.*

### 5. Frozen section of the optic nerve:

The harvested eyeballs were rinsed with PBS and fixed in paraformaldehyde solution overnight; the optic nerves were placed in 30% sucrose solution for dehydration at 4°C for approximately 1.5 days. The optic nerves were embedded using OCT embedding agent and fixed in a -80°C refrigerator. The frozen sectioning was performed at -20°C, and the thickness of optic nerve frozen sections was 8-10 µm. After washing off the OCT from the sections with PBS, anti-fluorescence attenuation sealer was added dropwise and a coverslip was placed. The entire optic nerve was completely photographed at 20× magnification using a confocal microscope.

Result statistics: the analysis was performed by using Image J software. A starting point was selected, and the number of axons at a distance extending from that starting point in the direction of weaker fluorescence was calculated. The results are shown in Figure 5D.

The results showed that relative to the G2S1K2+dox group, the G2S1K2 group can significantly inhibit RGC apoptosis due to crush injury (Figure 5C) and significantly promote axonal regeneration in the optic nerve (Figure 5D), suggesting that G2S1K2 has a senescence reprogramming effect.

## Claims

1. A composition for rejuvenating a cell, tissue, or organ of a subject, wherein the composition comprises:
i) any one factor selected from the group consisting of SOX1, SOX2, SOX3, RCOR2, GMNN, and GLIS1,
ii) any one factor selected from the group consisting of GATA1, GATA2, GATA3, GATA4, GATA6, FOXA2, SOX7, SOX17, NKX3.1, NR5A2, C/EBPα, OCT4, and PAX1, and
iii) factor Klf4 or Klf2.

2. The composition according to claim 1, **characterized in that**, the composition further comprises factor c-MYC.

3. The composition according to claim 2, **characterized in that**, the factor SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC is a protein, a nucleic acid encoding the same, or a mixture thereof,
preferably, the factor SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC is in the form of a protein, a fusion protein bound to a transmembrane domain, or a nucleic acid encoding the same.

4. The composition according to any one of claims 1 to 3, **characterized in that**, the factor SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC is in the form of an expression vector, wherein the expression vector is a plasmid vector, an add-on vector, a transposon, or a viral vector,
preferably, the viral vector is an adenoviral vector, an adeno-associated viral vector (AAV), a lentiviral vector, a Sendai virus vector, a cytomegalovirus vector, or a cowpox virus vector,
preferably, the factora are present on a same expression vector, or on a plurality of expression vectors.

5. The composition according to any one of claims 1 to 3, **characterized in that**, the factor SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC is in the form of DNA or RNA,
preferably, the factor is in the form of mRNA,
the factors are linked in tandem on a same mRNA molecule, or the factors SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, and/or c-MYC are in the form of a complex form of different mRNA molecules,
preferably, the factor is a synthetic mRNA, wherein the synthetic mRNA encodes a wild-type form, a mutant, or a genetically engineered form of SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC.

6. The composition according to any one of claims 1 to 3, **characterized in that**, the subject is a mammal including, but not limited to, human, mouse, rat, bovine, ovine, equine, canine, feline, porcine, or monkey,
the cell, tissue, or organ of the subject is derived from, but not limited to, eye, ear, nose, mouth including gum and root; bone, lung, breast, pancreas, stomach, esophagus; muscle including cardiac muscle; liver, blood vessel; skin including hair; heart, brain, neural tissue, kidney, testis, prostate, penis, cloaca, fin, ovary, or intestine.

7. The composition according to any one of claims 1 to 3, **characterized in that**, the factor SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC is a human protein or a non-human protein, or a protein having at least 80% identity to the amino acid sequence of the human protein or non-human protein and maintaining activity,
the non-human protein is a corresponding protein of mouse, rat, bovine, ovine, equine, canine, feline, porcine, monkey, chicken, duck, goose, or bird; or
the factor SOX1, SOX2, SOX3, RCOR2, GMNN, GATA1, GATA2, GATA3, GATA4, GATA6, FOXA2, SOX7, SOX17, NKX3.1, NR5A2, C/EBPα, OCT4, PAX1, Klf4, Klf2, GLIS1, or c-MYC is a nucleic acid encoding a human protein, or a nucleic acid encoding a non-human protein, or a nucleic acid having at least 80% identity to the nucleic acid sequence of a corresponding nucleic acid encoding the human protein or the non-human protein and maintaining activity,
the nucleic acid is a nucleic acid encoding a corresponding protein of mouse, rat, bovine, ovine, equine, canine, feline, porcine, monkey, chicken, duck, goose, or bird.

8. A method for rejuvenating a cell, tissue, or organ of a subject, **characterized in that**, the method comprises administering to the subject the composition of any one of claims 1 to 7.

9. Use of the composition of any one of claims 1 to 7 in the preparation of a medicament for rejuvenating a cell, tissue, or organ of a subject.

10. The method according to claim 8 or the use according to claim 9, **characterized in that**, the rejuvenating a cell, tissue, or organ of a subject comprises restoring the lost transcriptional profile or epigenetic information due to aging, injury, disease or combination thereof in at least one cell, tissue, or organ of the subject.

11. The method according to claim 8 or the use according to claim 9, **characterized in that**, the rejuvenating a cell, tissue, or organ of a subject comprises restoring the function of the cell, increasing the potential of the cell, enhancing the viability of the cell, or increasing the replicative capacity or lifespan of the cell, or a combination thereof.

12. The method according to claim 8 or the use according to claim 9, **characterized in that**, the rejuvenating a cell, tissue, or organ of a subject comprises promoting axonal regeneration of a neuronal cell in a subject; reversing the epigenetic age of a skin fibroblast in a subject; reversing senescence of a skin fibroblast in a subject; reversing senescence of a chondrocyte in a subject; reversing senescence of a muscle stem cell in a subject; promoting improvement of osteoarthritis symptoms in a subject; or promoting improvement of ocular disease symptoms in a subject.

13. A kit comprising an inducing factor for rejuvenating somatic cells in a subject, **characterized in that**, the inducing factor comprises:
i) any one factor selected from the group consisting of SOX1, SOX2, SOX3, RCOR2, GMNN, and GLIS1,
ii) any one factor selected from the group consisting of GATA1, GATA2, GATA3, GATA4, GATA6, FOXA2, SOX7, SOX17, NKX3.1, NR5A2, C/EBPα, OCT4, and PAX1, and
iii) factor Klf4 or Klf2.

14. The kit according to claim 13, **characterized in that** the inducing factor further comprises c-MYC.

15. The composition of any one of claims 1 to 7, the method according to claim 8, the use according to claim 9, the method or use according to any one of claims 10 to 12, or the kit according to any one of claims 13 to 14, **characterized in that**, the subject suffers from, is suspected of suffering from, or is at risk of suffering from:
a disease, condition, or symptom associated with impaired cell, tissue, or organ viability, or with aging;
the disease, condition, or symptom associated with impaired cell, tissue, or organ viability, or with aging is a neurodegenerative disease, a cardiovascular disease, a metabolic disease, a musculoskeletal disease, an inflammatory disease, a symptom or condition associated with skin, an ocular disease, a fibrotic disease, or a disease associated with reproductive system;
preferably, the neurodegenerative disease is stroke, Huntington's chorea, dementia, Alzheimer's disease, or Parkinson's disease;
preferably, the cardiovascular disease is hypertension, coronary arrhythmia, heart disease, hypotension, heart failure, angina pectoris, arteriosclerosis, myocardial necrosis, myocarditis, congestive heart failure, atrioventricular block, atherosclerosis, or myocardial infarction;
preferably, the metabolic disease is type I diabetes, type II diabetes, hyperglycemia, hyperinsulinemia, insulin resistance, obesity, or gout;
preferably, the musculoskeletal disease is muscular dystrophy, myasthenia, myasthenia gravis, osteoporosis, osteoarthritis, osteochondrosis, osteochondritis, osteonecrosis, or osteopenia;
preferably, the inflammatory disease is systemic lupus erythematosus, rheumatic polymyalgia, gouty arthritis, degenerative arthritis, rheumatoid arthritis, inflammatory arthritis, Hashimoto's thyroiditis, inflammatory bowel disease, hepatitis, pneumonia, respiratory inflammation, or encephalitis;
preferably, the symptom or condition associated with the skin is wrinkles, age spots, seborrheic keratosis, tinea manuum and pedis, urticaria, neurodermatitis, or pigmented nevi;
preferably, the ocular disease is macular degeneration, retinal degeneration, retinal detachment, diabetic retinopathy, glaucoma, optic atrophy, floaters, cataract, non-arteritic anterior ischemic optic neuropathy, chorioretinitis, retinitis pigmentosa, or optic nerve injury;
preferably, the fibrotic disease is pulmonary fibrosis, hepatic fibrosis, myocardial fibrosis, renal interstitial fibrosis, myelofibrosis, pleural fibrosis, or intestinal wall fibrosis;
preferably, the disease associated with the reproductive system is erectile dysfunction or premature ovarian failure.
